(19) Europäisches Patentamt
European Patent Office
Office européen des brevets

(11) Publication number: **0 416 740 A2**

# (12) EUROPEAN PATENT APPLICATION

(21) Application number: **90308501.7**

(51) Int. Cl.5: **C07K 5/02**, A61K 37/64

(22) Date of filing: **01.08.90**

The title of the invention has been amended (Guidelines for Examination in the EPO, A-III, 7.3).

(30) Priority: **08.08.89 GB 8918074**
**08.12.89 GB 8927873**
**08.12.89 GB 8927874**

(43) Date of publication of application:
**13.03.91 Bulletin 91/11**

(84) Designated Contracting States:
**AT BE CH DE DK ES FR GB GR IT LI LU NL SE**

(71) Applicant: **Beecham Group p.l.c.**
**SB House Great West Road**
**Brentford Middlesex TW8 9BD(GB)**

(72) Inventor: **Smith, Stephen Allan SmithKline Beecham Pharmaceuticals Coldharbour Road The Pinnacles**
**Fourth Avenue Harlow Essex CM19 5AD(GB)**
Inventor: **Nash, David John SmithKline Beecham Pharmaceuticals Coldharbour Road The Pinnacles**
**Fourth Avenue Harlow Essex CM19 5AD(GB)**
Inventor: **Ham, Peter SmithKline Beecham Pharmaceuticals Coldharobour Road The Pinnacles**
**Fourth Avenue Harlow Essex CM19 5AD(GB)**

(74) Representative: **Jones, Pauline et al**
**SmithKline Beecham, Corporate Patents,**
**Great Burgh, Yew Tree Bottom Road**
**Epsom, Surrey KT18 5XQ(GB)**

## (54) Novel compounds with renin-inhibiting activity.

(57) Compounds of formula (I), and pharmaceutically acceptable salts thereof:

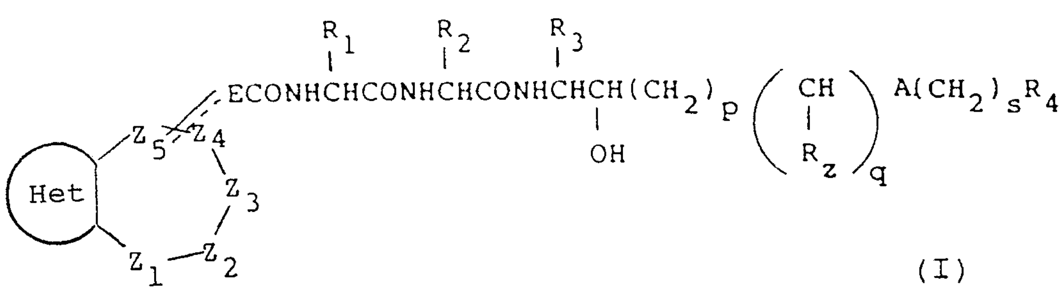

(I)

wherein
the Het ring is of sub-formula (a) or (b):

EP 0 416 740 A2

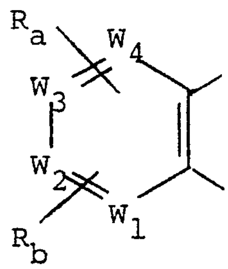

(a)

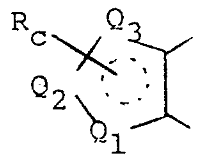

(b)

wherein

one or two of $W_1$, $W_2$, $W_3$ and $W_4$ is N or NO (such that if two are NO then these are $W_1$ and $W_4$), and the others are CH, $CR_a$ or $CR_b$;

one of $Q_1$, $Q_2$ and $Q_3$ is S and the other two are CH and $CR_c$;

either $Z_1$ and $Z_2$ are absent and $Z_3$, $Z_4$ and $Z_5$ and the carbon atoms to which $Z_3$ and $Z_5$ are attached, form a 5-membered non-aromatic heterocyclic ring; or

$Z_1$ is absent and $Z_2$, $Z_3$, $Z_4$, $Z_5$ and the carbon atoms to which $Z_2$ and $Z_5$ are attached, form a 6-membered non-aromatic heterocyclic ring; or

$Z_1$, $Z_2$, $Z_3$, $Z_4$ and $Z_5$ and the carbon atoms to which $Z_1$ and $Z_5$ are attached, form a 7-membered non-aromatic heterocyclic ring;

E is absent or is $(CH_2)_n$ or $CH(CH_2)_{n-1}$ wherein n is 1 to 4;

A is -CONH-, -NHCO-, -COO-, -S(O)r- wherein r is 0, 1 or 2, or $-CH_2-$;

p is 0, 1 or 2;

s is 0, 1, 2, 3 or 4;

q is 0 or 1;

$R_z$ is hydrogen, $C_{1-6}$ alkyl or, when A is $-CH_2-$, hydroxy;

$R_a$ and $R_b$ are independently selected from hydrogen or a substituent;

$R_1$ is $CH_2R_9$ wherein $R_9$ is optionally substituted aryl or heteroaryl;

$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;

$R_3$ is $CH_2R_{12}$ wherein $R_{12}$ is $C_{1-6}$ alkyl, $C_{3-8}$cycloalkyl or phenyl;

$R_4$ is $C_{1-6}$ alkyl, $C_{3-8}$ cycloalkyl, a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, or $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and

the dashed line represents an optional bond (when E is present);

which are renin inhibitors, a process for their preparation and their use as pharmaceuticals.

## NOVEL COMPOUNDS

The invention relates to novel compounds having pharmacological activity, to processes for their preparation, to pharmaceutical preparations containing them and to their use in medicine.

Renin is a natural enzyme, disorders in relation to which are implicated in many cases of hypertension. It is released into the blood from the kidney, and cleaves from a blood glycoprotein a decapeptide known as angiotensin-I. Circulating angiotensin-I is cleaved in plasma, and in lung, kidney and other tissues to an octapeptide, angiotensin-II, which raises blood pressure both directly by causing arteriolar constriction and indirectly by stimulating release of the sodium-retaining hormone aldosterone from the adrenal gland and thus causing a rise in extracellular fluid volume. The latter effect is caused by angiotensin-II itself or a heptapeptide cleavage product angiotensin-III.

Inhibitors of renin have been described as useful in the treatment of hypertension.

A group of compounds has now been discovered, which inhibit the enzyme renin and therefore have potential blood pressure lowering activity, useful in the treatment of hypertension. Compounds having an associated mechanism of action have also been described as possessing anti-retroviral activity and the present compounds may therefore be of potential use in the treatment of diseases caused by retroviruses including human immunodeficiency virus (HIV-1 and 2) and HTLV I and II. They may also be of potential use in the treatment of other cardiovascular disorders, such as congestive heart failure, and have beneficial effects on learning and memory and mood elevation activity, of potential use in the treatment of CNS disorders such as Alzheimer's disease and depression; they may also be of potential use in the treatment of glaucoma.

Accordingly, the present invention provides a compound of formula (I), or a pharmaceutically acceptable salt thereof:

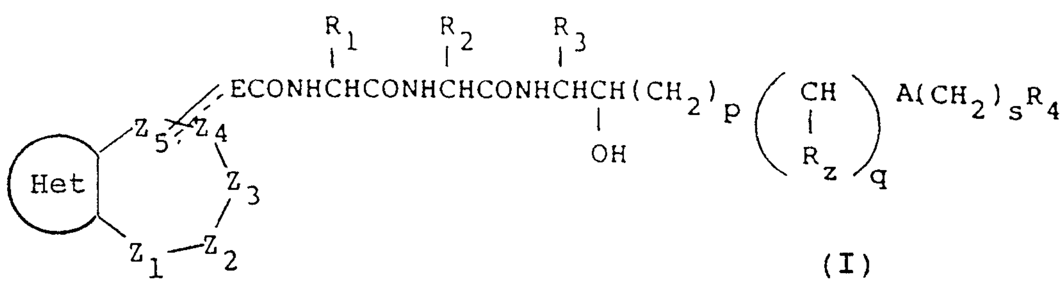

(I)

wherein

the Het ring is of sub-formula (a) or (b):

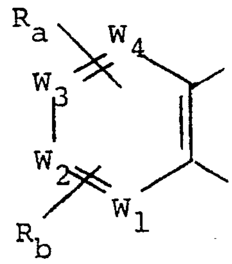

(a)

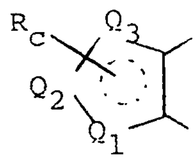

(b)

wherein

one or two of $W_1$, $W_2$, $W_3$ and $W_4$ is N or NO (such that if two are NO then these are are $W_1$ and $W_4$), and the others are CH, $CR_a$ or $CR_b$;

one of $Q_1$, $Q_2$ and $Q_3$ is S and the other two are CH and $CR_c$;

either $Z_1$ and $Z_2$ are absent and $Z_3$, $Z_4$ and $Z_5$ and the carbon atoms to which $Z_3$ and $Z_5$ are attached, form a 5-membered non-aromatic heterocyclic ring; or

$Z_1$ is absent and $Z_2$, $Z_3$, $Z_4$, $Z_5$ and the carbon atoms to which $Z_2$ and $Z_5$ are attached, form a 6-membered non-aromatic heterocyclic ring; or

$Z_1$, $Z_2$, $Z_3$, $Z_4$ and $Z_5$ and the carbon atoms to which $Z_1$ and $Z_5$ are attached, form a 7-membered non-aromatic heterocyclic ring;

E is absent or is $(CH_2)_n$ or $CH(CH_2)_{n-1}$ wherein n is 1 to 4;

A is -CONH- -NHCO-, -COO-, -S(O)r- wherein r is 0, 1 or 2, or $-CH_2-$;

p is 0, 1 or 2;

s is 0, 1, 2, 3 or 4;

q is 0 or 1;

$R_z$ is hydrogen, $C_{1-6}$ alkyl or, when A is $-CH_2-$, hydroxy;

$R_a$ and $R_b$ are independently selected from hydrogen or a substituent;

$R_1$ is $CH_2R_9$ wherein $R_9$ is optionally substituted aryl or heteroaryl;

$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-5}$ alkoxycarbonylamino;

$R_3$ is $CH_2R_{12}$ wherein $R_{12}$ is $C_{1-6}$ alkyl, $C_{3-8}$ cycloalkyl or phenyl;

$R_4$ is $C_{1-6}$ alkyl, $C_{3-8}$ cycloalkyl, a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, or $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and

the dashed line represents an optional bond (when E is present).

When the Het ring is of sub-formula (a)

Often $W_3$ or $W_4$ is N or NO, usually $W_3$ is N when E is attached at $Z_5$, or $W_1$ is N when E is attached at $Z_5$.

When the Het ring is of sub-formula (b)

Often $Q_1$ or $Q_3$ is S.

When $Z_1$ and $Z_2$ are absent:

Values of $Z_3$, $Z_4$ and $Z_5$ include wherein one of $Z_3$, $Z_4$ and $Z_5$ is attached to E and is N, CH or C (wherein the optional exocyclic bond is present); the second of $Z_3$, $Z_4$ and $Z_5$ is O, S, SO, $SO_2$ or NR wherein R is hydrogen or $C_{1-6}$ alkyl; and the third is O, S, SO, $SO_2$, NR, $CH_2$ or C=O provided that, one of $Z_3$ and $Z_4$, and one of $Z_4$ and $Z_5$, is $CH_2$, CH or C when the other is S or O, or is $CH_2$, CH, C, NR or N when the other is SO or $SO_2$; or $Z_4$ is SO, $SO_2$ or C=O when one of $Z_3$ and $Z_5$ is N and the other is O, S or NR; or $Z_3$ is CO, $Z_4$ is O and $Z_5$ is CH.

Examples of $Z_3$, $Z_4$ and $Z_5$ then include

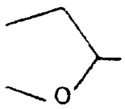 , 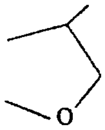 , 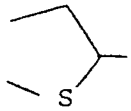 , 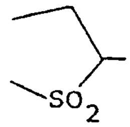 ,

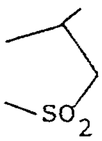 , 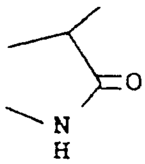 , 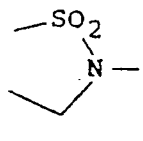 , 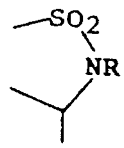 ,

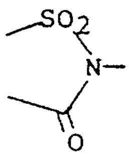 , 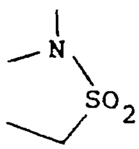 , 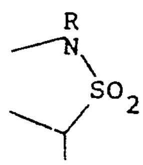 , 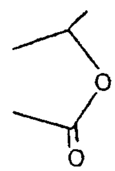 , ,

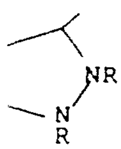 , 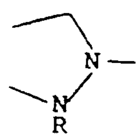 , 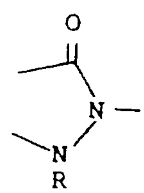 , 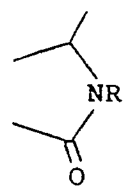 ,

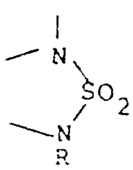 , 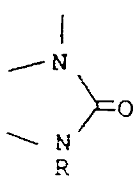 , 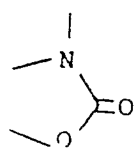 , 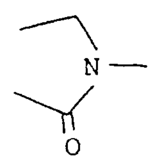

Preferably $Z_3$ is CO, $Z_4$ is N, $Z_5$ is CH and E is attached at $Z_4$.

<u>When $Z_1$ is absent:</u>

Values of $Z_2$, $Z_3$, $Z_4$ and $Z_5$ include wherein one of $Z_2$, $Z_3$, $Z_4$ and $Z_5$ is attached to E and is N, CH or C (wherein the optional exocyclic bond is present); another of $Z_2$, $Z_3$, $Z_4$ and $Z_5$ is O, S, SO, $SO_2$ or NR wherein R is hydrogen or $C_{1-6}$ alkyl; another of $Z_2$, $Z_3$, $Z_4$ and $Z_5$ is O, S, SO, $SO_2$, NR or $CH_2$; and the other of $Z_2$, $Z_3$, $Z_4$ and $Z_5$ is S, SO, $SO_2$, NR, $CH_2$ or C=O provided that, one of $Z_2$ and $Z_3$, one of $Z_3$ and $Z_4$, or one of $Z_4$ and $Z_5$ is $CH_2$, CH or C when the other is S or O, or is $CH_2$, CH, C, NR or N when the other is SO or $SO_2$; or $Z_3$/$Z_4$ is SO, $SO_2$ or C=O when one of $Z_2$/$Z_3$ and $Z_4$/$Z_5$ is N or NR and the other is O, S, N or NR; or $Z_2$-$Z_3$-$Z_4$-$Z_5$ is -NRCOCON< or >NCOCONR-; or $Z_2$/$Z_5$ is CO, $Z_3$/$Z_4$ is O and $Z_4$,$Z_5$/$Z_2$,$Z_3$ are CH and $CH_2$.
i.e. the following are excluded in $Z_2$ to $Z_5$:-

i) adjacent same heteroatoms (O, S, N) except N-N; but no N-N-N;
iii) $SO_X$-$SO_Y$ or O-$S_y$ (x and y are 0-2);
iv) O-C-O, S-C-O, S-C-S;
v) CO-SO or CO-$SO_2$;
vi) two heteroatoms (O, S, N) separated by a Z when CO unless one of them is N;
vii) more than two of $Z_2$ to $Z_5$ is CO.

Examples of $Z_2$, $Z_3$, $Z_4$ and $Z_5$ then include

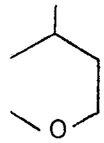 , 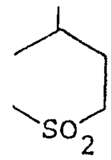 , 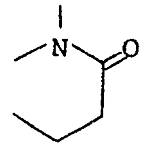 ,

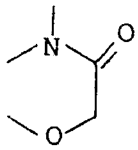 , 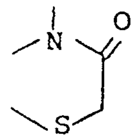 , 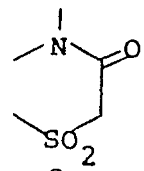 ,

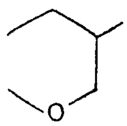 , 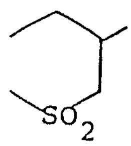 , 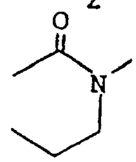 ,

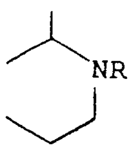 , 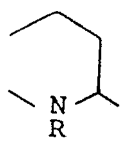 , 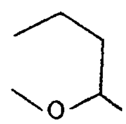 ,

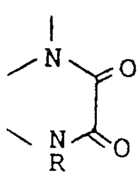 , 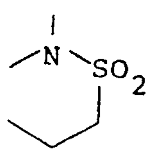 , 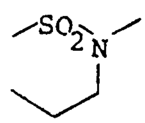 ,

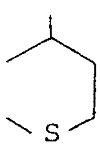 , 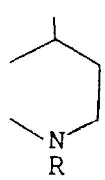 , 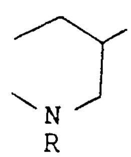 ,

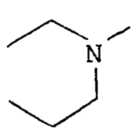

When $W_3$ is N and the other W are CH, $CR_a$ or $CR_b$, often $Z_2$ is S, $Z_3$ is $CH_2$, $Z_4$ is CO, $Z_5$ is N and E is attached at $Z_5$.

<u>When $Z_1$ and $Z_2$ are present</u>

Heterocyclic ring heteroatoms for $Z_1$ to $Z_5$ are selected from oxygen, sulphur, SO, $SO_2$, nitrogen or N-alkyl. The ring may include unsaturation (C=C) or a carbon attached to exocyclic oxo (C=O).

Examples of $Z_1$, $Z_2$, $Z_3$, $Z_4$ and $Z_5$ then include

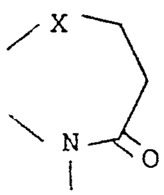 , 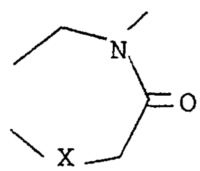 , 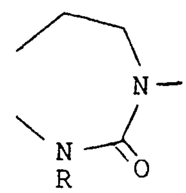 , 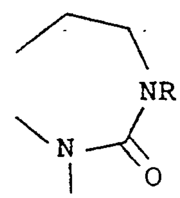

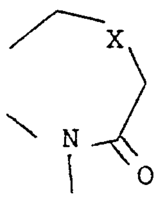 , 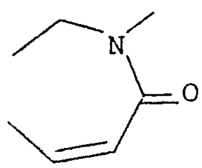 , 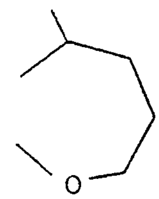

wherein X is O, S, SO, $SO_2$, NH, N-$C_{1-6}$ alkyl or $CH_2$.

Preferably E is attached at a nitrogen atom.

Suitable examples of $R_a$ and $R_b$ in sub-formula (a), include a moiety selected from halogen, $C_{1-6}$ alkyl, $C_{1-6}$ alkoxy, cyano, CHO, $C_{1-6}$ alkyl substituted by optionally substituted amino or $R_a/R_b$ is a group $NR_5R_6$, $CO_2R_5$, wherein $R_5$ is hydrogen, $C_{1-6}$ alkyl or optionally substituted benzyl and $R_6$ is hydrogen or $C_{1-6}$ alkyl.

When one of $R_a$ and $R_b$ is hydrogen and the other is a halogen substituent, the substituent is preferably in the 3-position relative to the ring nitrogen atom, when one of $W_1$ to $W_4$ is N or NO.

Suitable examples of $R_c$ in sub-formula (b), include a moiety selected from $C_{1-6}$ alkyl, optionally substituted amino, cyano, $C_{1-6}$ alkyl substituted by optionally substituted amino or $R_c$ is $CO_2R_5$ wherein $R_5$ is hydrogen, $C_{1-6}$ alkyl or optionally substituted benzyl.

Suitable examples of substituents in optionally substituted amino groups in $R_a/R_b/R_c$ then include one or two groups independently selected from $C_{1-6}$ alkyl or $C_{1-6}$ alkyl substituted by carboxy or $C_{1-6}$ alkoxycarbonyl.

Alternatively, $NR_5R_6$ or other substituted amino groups in $R_a/R_b/R_c$ may be pyrrolidinyl, piperidinyl, morpholinyl or piperazinyl; optionally N-substituted by $C_{1-6}$ alkyl, or as an N-oxide thereof.

Preferably one of $R_a$ and $R_b$ is hydrogen and the other is $CH_2NH_2$, $CO_2H$ or $CH_2NHCH_2CO_2H$.

$R_c$ is preferably hydrogen.

In regard to values of A and p and q, reference is hereby made to the following literature:-

1. J. Med. Chem. 1988, 31 , 1918
(p = 1, q = 0, A = -CONH-)
2. J. Med. Chem. 1987, 30 , 1224
(p = 2, q = 0, A = -NHCO-)
3. J. Med. Chem. 1987, 31 , 701
(p = O, q = 0, A = -COO-)
4. J. Med. Chem. 1987, 31 , 1839
(p = 1, q = 1, A = -CONH-)
5. J. Med. Chem. 1987, 30 , 1729 and refs. therein
(p = 2, q = 1, A = $S(O)_r$)

6. WO 88/05050
($p = 0, q = 1, A = CH_2, R_z = OH$)
7. EP-A-172346
($p = 0, q = 1, A = CH_2, R_z = H$)

Suitable values of $R_9$ and $R_{11}$ when aryl include phenyl or naphthyl and when heteroaryl, include a 5- or 6-membered monocyclic or 9- or 10- membered bicyclic of which 5- or 6- membered monocyclic heteroaryl is preferred. In addition, 5- or 6-membered monocyclic or 9- or 10-membered bicyclic heteroaryl preferably contains one, two or three heteroatoms which are selected from the class of oxygen, nitrogen and sulphur and which, in the case of there being more than one heteroatom, are the same or different. Examples of 5-or 6-membered monocyclic heteroaryl containing one, two or three heteroatoms which are selected from the class of oxygen, nitrogen and sulphur include furyl, thienyl, pyrryl, oxazolyl, thiazolyl, imidazolyl and thiadiazolyl, and pyridyl, pyridazyl, pyrimidyl, pyrazolyl and triazolyl. Preferred examples of such groups include furanyl, thienyl, pyrryl and pyridyl, in particular 2- and 3-furanyl, 2- and 3-pyrryl, 2- and 3-thienyl, and 2-, 3- and 4-pyridyl. Examples of 9- or 10-membered bicyclic heteroaryl containing one, two or three heteroatoms which are selected from the class of oxygen, nitrogen and sulphur include benzofuranyl, benzothienyl, indolyl and indazolyl, quinolyl and isoquinolyl, and quinazolyl. Examples of such groups include 2- and 3-benzofuranyl, 2- and 3-benzothienyl, and 2- and 3-indolyl, and 2- and 3-quinolyl and, for $R_{11}$, 4-benzimidazolyl.

Suitable examples of groups or atoms for optional substitution of $R_5$ when benzyl and $R_9$ and $R_{11}$ include one, two or three substituents independently selected from $C_{1-4}$ alkyl, $C_{1-4}$ alkoxy, halo (such as fluoro, chloro, bromo), hydroxy, nitro, and cyano.

Preferred examples of $R_9$ include phenyl and naphthyl, and preferred examples of $R_{11}$ when aryl or heteroaryl include phenyl, imidazol-4-yl and -2-yl, pyrazol-1-yl and 4-methylpyrazol-1-yl.

Preferably the amino acid residue containing $R_2$ is Leu, β-pyrazolylalanine or His.

Abbreviations which may be used herein are as follows:

| Amino Acid Residue | Abbreviations |
|---|---|
| histidine | His |
| isoleucine | Ile |
| leucine | Leu |
| 1-naphthylalanine | NAla |
| norleucine | Nle |
| phenylalanine | Phe |

The α-amino acid components are in the (S)-configuration (or L-form).

In a particular aspect, the present invention provides a compound of formula (IA) or a pharmaceutically acceptable salt thereof:

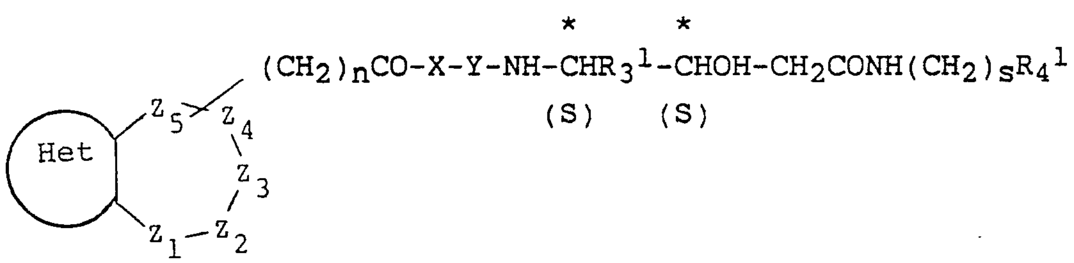

(IA)

wherein
X is Phe;
Y is Leu or His;

$R_3^1$ is cyclohexylmethyl;

$R_4^1$ is $C_{4-5}$ alkyl and s is 0 or s is 2 to 4 and $R_4^1$ is carboxy or a saturated or unsaturated heterocyclic ring; and

the remaining variables are as defined in formula (I).

Suitable values for alkyl groups in $R_a$, $R_b$, $R_z$, R and $R_2$ to $R_{12}$ in formulae (I) and (IA) include methyl, ethyl, n - and iso -propyl, n -, sec -, iso - and tert -butyl, n -, iso -, sec -, tert - and neo -pentyl. $C_{3-8}$ cycloalkyl groups are cyclopropyl, cyclobutyl, cyclopentyl, cyclohexyl, cycloheptyl and cyclooctyl.

Suitable values for $R_a$ and $R_b$ halo include fluoro, chloro, bromo and iodo, preferably chloro or bromo.

Preferably $R_4$ or $R_4^1$ when alkyl is iso -butyl.

Suitable examples of $R_4$ when monocyclic heteroaryl include those listed for $R_9$ and $R_{11}$, preferably 3-pyridyl or an N-oxide thereof, or 1- or 2-imidazolyl.

When $R_4$ is a saturated heterocyclic ring, suitable examples include piperidinyl, pyrrolidinyl, piperazinyl and morpholinyl; optionally N-substituted by $C_{1-6}$ alkyl group(s) or as an N-oxide thereof.

The right hand sequence in formula (I) and (IA) is preferably 4(S)-amino-5-cyclohexyl-3(S)-hydroxypentanoic acid (ACHPA), from the amino acid containing $R_3$ or $R_3^1$ to $NH(CH_2)_sR_4$.

Preferably n is 1, 2, 3 or 4.

When $Z_1$ is absent, E is favourably attached at the 4-position with respect to $Z_2$ when n = 2, and at the 3-position when n>2. When $Z_1$ and $Z_2$ are absent, E is preferably at the 3-position with respect to $Z_3$ when n = 2, and at the 2-position when n>2. When $Z_1$ and $Z_2$ are present, E is usually attached at a nitrogen atom or at $Z_1$ or $Z_5$ when a carbon atom. E is preferably attached at a nitrogen atom.

Pharmaceutically acceptable salts include acid addition salts which may be, for example, salts with inorganic acids such, for example, as hydrochloric acid, hydrobromic acid, orthophosphoric acid or sulphuric acid, or organic acids such, for example, as methanesulphonic acid, toluenesulphonic acid, acetic acid, trifluoroacetic acid, propionic acid, lactic acid, citric acid, tartaric acid, fumaric acid, malic acid, succinic acid, salicylic acid or acetylsalicylic acid.

Pharmaceutically acceptable salts may also include alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine.

It will be appreciated that the compounds of the invention may exist as solvates, such as hydrates, and these are included whenever, a compound of formula (I) or a salt thereof, is herein referred to.

The compounds of formula (I) wherein E is attached to $Z_1$(when present)/$Z_2$(when present)/$Z_3$/$Z_4$/$Z_5$ when CH, and (IA) have at least one asymmetric centre in addition to those attached to $R_1$ and $R_2$ (in X and Y) and $R_3$ and those indicated in formula (IA), and are therefore capable of existing in more than one stereoisomeric form. The invention extends to each of these forms and to mixtures thereof. Preferably the configuration at the carbon atoms bearing $R_1$, $R_2$ and $R_3$ is the (S)-configuration.

It will be appreciated that, when the optional bond depicted in formula (I) is present, the compounds are capable of existing in E and Z ( trans and cis ) forms.

The invention extends to each of these forms and to mixtures thereof.

The compounds of the invention are preferably in pharmaceutically acceptable form. By pharmaceutically acceptable form is meant, inter alia, of a pharmaceutically acceptable level of purity excluding normal pharmaceutical additives such as diluents and carriers, and including no material considered toxic at normal dosage levels. A pharmaceutically acceptable level of purity will generally be at least 50% excluding normal pharmaceutical additives, preferably 75%, more preferably 90% and still more preferably 95%.

A compound of the invention may be prepared by those methods known in the art for the synthesis of compounds of analogous structure, such as peptides, and in this regard reference is made, by way of illustration only, to the literature reference: S.R. Pettit, " Synthetic Peptides ", (Elsevier Scientific Publishing Co. 1976).

The present invention also provides a compound of the present invention which has been prepared synthetically.

A compound of the present invention may, for example, be formed by the sequential coupling of appropriate amino acids with the acid of formula (II):

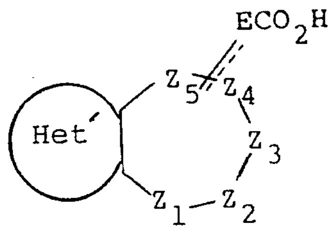

(II)

wherein Het′ is Het as defined in formula (I), but substituted by $R_a'$, $R_b'$ in sub-formula (a) and by $R_c'$ in sub-formula (b), wherein $R_a'$, $R_b'$ and $R_c'$ are $R_a$, $R_b$ and $R_c$ respectively or groups or atoms convertible thereto; or by the initial preparation and subsequent coupling of peptide subunits with the acid of formula (II), the subunits themselves prepared in stepwise manner; in either case classical solution chemistry methods analogous to those used in peptide synthesis, may be employed.

The coupling reactions may be effected by, for example, activating the reacting carboxyl group of the acid of formula (II) or amino acid, and reacting this with the amino group of the substrate unit. Details of suitable, optional activating and protecting (masking) groups and of suitable reaction conditions (for the coupling reactions and for the introduction and removal of protecting groups) giving, preferably, the minimum of racemisation, may be found in the above-referenced literature.

It will be appreciated that, when A is other than -CONH- or -COO-, the C-terminus group is other than an amino acid, in which case the coupling is carried out with an appropriate other precursor, as described in the aforementioned literature -references 2., 5., 6. and 7.

Accordingly, the present invention further provides a process for the preparation of a compound of formula (I) or a pharmaceutically acceptable salt thereof which comprises reacting a reagent of the formula (III):

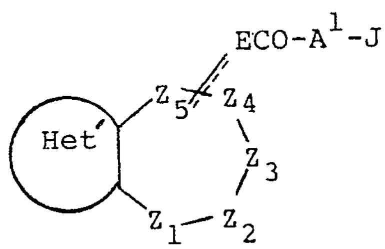

(III)

wherein Het′ is Het as defined but wherein $R_a$, $R_b$ and $R_c$ are $R_a'$, $R_b'$ and $R_c'$, which are $R_a$, $R_b$ and $R_c$ respectively or groups or atoms convertible thereto; $A^1$ is absent or represents an appropriate amino acid or dipeptide unit; J is OH or a leaving group; and the remaining variables are as hereinbefore defined; with a reagent of the formula (IV):

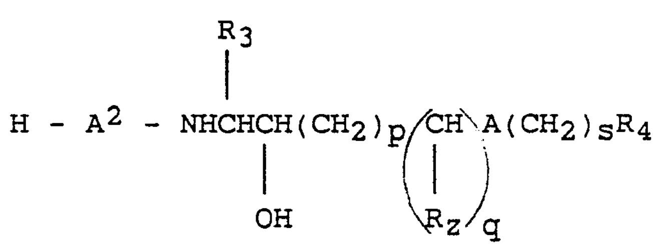

(IV)

wherein

$A^2$ is absent or represents an appropriate amino acid or dipeptide unit, such that $A^1$ + $A^2$ is $-NHCHR_1CONHCHR_2CO-$, and the remaining variables are as hereinbefore defined; and thereafter, if

desired or necessary deprotecting (within $A^1$ or $A^2$ of the products, and/or converting $Z_1$, $Z_2$, $Z_3$, $Z_4$ or $Z_5$ to other $Z_1$, $Z_2$, $Z_3$, $Z_4$ or $Z_5$, $R_a'/R_b'/R_c'$ in Het' to $R_a$, $R_b$ and/or $R_c$ and/or $W_1$, $W_2$, $W_3$ or $W_4$ in Het' when N to NO; and/or forming a pharmaceutically acceptable salt thereof.

Suitable examples of J when a leaving group include halo, such as chloro or bromo, and other suitable groups which are displaceable by an amino nucleophile, such as $C_{1-6}$ alkoxycarbonyloxy.

It is generally preferred, especially when $A^1$ is not absent, however, that J is OH, and a suitable coupling reagent or dehydrating catalyst is used to effect the reaction, such as N,N-dicyclohexylcarbodiimide and those described in the Descriptions and Examples hereinafter.

Deprotection of $A^1$ and/or $A^2$ takes place conventionally, in accordance with the particular protecting group(s) employed.

Pharmaceutically acceptable salts may be formed conventionally.

$Z_1$, $Z_2$, $Z_3$, $Z_4$ or $Z_5$ when S or SO may be converted to SO or $SO_2$ respectively (or S to $SO_2$) by conventional oxidation methods, preferably in a non-aqueous solvent, such as a chlorinated hydrocarbon, in the presence of an organic peracid, such as 3-chloroperoxybenzoic acid, or in water in the presence of a soluble strong inorganic oxidant, such as an alkali metal permanganate or with aqueous hydrogen peroxide. A particularly suitable alternative oxidation method involves the use of magnesium monoperphthalate, as described in Description 10b) hereinafter.

It will be apparent that compounds of the formula (I) containing a Het' ring wherein the $R_a'/R_b'/R_c'$ group is other than $R_a/R_b/R_c$ and which is convertible to an $R_a/R_b/R_c$ group, are useful novel intermediates. A number of such conversions is possible, not only for the final product compounds of formula (I) when $R_a'/R_b'/R_c'$ are other than $R_a/R_b/R_c$, but also within $R_a/R_b/R_c$, and also for their intermediates as follows:

(i) an $R_a/R_b$ nitro substituent is convertible to an amino substituent by reduction;

(ii) a $C_{1-7}$ acylamino substituent is convertible to an amino substituent by deacylation;

(iii) a hydrogen substituent in sub-formula (a) is convertible to a halogen substituent by halogenation;

(iv) an amino substituent is convertible to a corresponding substituent which is substituted by one or two alkyl groups, by N-alkylation;

(v) a cyano substituent may be converted to an aminomethyl substituent by reduction;

(vi) a bromo substituent may be converted to a cyano substituent by reaction with copper(I) cyanide.

Conversions (i) to (vi) are only exemplary and are not exhaustive of the possibilities. It will be appreciated that it is often desirable to carry out these conversions at an earlier stage, in the intermediate acid of formula (II).

In regard to (i), the reduction is carried out with a reagent suitable for reducing nitroanisole to amincanisole.

In regard to (ii), deacylation is carried out by treatment with a base, such as an alkali metal hydroxide.

In regard to (iv), alkylation is carried out with a corresponding alkylating agent such as the chloride or bromide under conventional conditions.

In regard to (v), the reduction takes place by reaction with sodium borohydride/cobalt chloride or platinum oxide/hydrogen.

In regard to (vi), the reaction takes place under conventional conditions.

Conversion of $W_1$, $W_2$, $W_3$ or $W_4$ when N to NO may be achieved by similar oxidation methods to those used for $Z_1/Z_2/Z_3/Z_4/Z_5$ when S or SO to SO or $SO_2$, such as using 3-chloroperoxybenzoic acid. Selective oxidation may be achieved by an appropriate choice of oxidising agent and conditions.

Compounds of the general formulae (II) and (III) may themselves be prepared by standard techniques analogous to those described above.

The acids of formula (II) are either known compounds or are prepared by analogous methods to these and for structurally similar known compounds.

The preparation of the amino acid of formula (V):

$$H_2N-\overset{*}{C}HR_3{}^1-\overset{*}{C}HOH-CH_2CO_2H \qquad (V)$$

$$\quad (S) \quad (S)$$

is described in J. Med. Chem 1985, 28 , 1779-1790.

As regards the appropriate corresponding intermediates to those of formula (V), including those wherein A is other than -CONH-, reference is hereby made to the literature references 2. to 7. listed hereinbefore.

It will be appreciated that protected forms of compounds of the present invention are novel intermediates and form an aspect of the invention.

Particularly suitable methods of preparation of the compounds of the present invention are as described in the Descriptions and Examples hereinafter. The couplings may be carried out sequentially beginning by coupling the acid of formula (II) with, for example, phenylalanine or 1-naphthylalanine, followed by coupling with Y, for example, leucine or histidine, and finally, coupling with the amino acid of formula (V). In a preferred aspect, however, either the acid of formula (II) is coupled with a tripeptide unit formed between the amino acid of formula (V); leucine, histidine or other $R_2$ containing amino acid; and phenylalanine or naphthylalanine; or alternatively the acid of formula (II) is coupled with phenylalanine or naphthylalanine and this is coupled with a dipeptide unit formed between the $R_2$ containing amino acid and the amino acid of formula (V).

As mentioned previously the compounds of the invention have been found to be renin inhibitors, and therefore they are of potential use in the treatment of hypertension. They may also be of potential use in the other diseases and disorders hereinbefore referred to.

The present invention also provides a pharmaceutical composition which comprises a compound of formula (I) or a pharmaceutically acceptable salt thereof, and a pharmaceutically acceptable carrier. In particular, the present invention provides an anti-hypertensive pharmaceutical composition which comprises an anti-hypertensive effective amount of a compound of formula (I) or a pharmaceutically acceptable salt thereof, and a pharmaceutically acceptable carrier.

The compositions are preferably adapted for oral administration. However, they may be adapted for other modes of administration, for example parenteral administration for patients suffering from heart failure. Other alternative modes of administration include sublingual or transdermal administration, or for example, eye-drop compositions, for the treatment of glaucoma.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

For transdermal administration, formulations suitable for topical use may be employed, optionally containing penetration enhancers.

The compositions may contain from 0.1% to 99% by weight, preferably from 10-60% by weight, of the

active material, depending on the method of administration.

The present invention further provides a method of prophylaxis or treatment of hypertension in mammals including man, which comprises administering to the suffering mammal an anti-hypertensively effective amount of a compound of formula (I) or a pharmaceutically acceptable salt thereof.

An effective amount will depend on the relative efficacy of the compounds of the present invention, the severity of the hypertension being treated and the weight of the sufferer. However, a unit dose form of a composition of the invention may contain from 0.1 to 500 mg of a compound of the invention and more usually from 1 to 100 mg, for example 2 to 50 mg such as 2, 3, 4, 5, 10, 20 or 30mg. Such compositions may be administered from 1 to 6 times a day, more usually from 2 to 4 times a day, in a manner such that the daily dose is from 1 to 1000mg for a 70 kg human adult and more particularly from 5 to 500 mg.

No toxicological effects are indicated at the aforementioned dosage ranges.

The present invention further provides a compound of formula (I) or a pharmaceutically acceptable salt thereof for use in the treatment or prophylaxis of hypertension.

The following Descriptions relate to the preparation of intermediates and the following Examples relate to the preparation of compounds of formula (I).

The following tabulations show the structures prepared.

(* indicates the point of attachment of E).

In the following, the abbreviations used are:

| Abbreviation | Definition |
|---|---|
| ACHPAA | 4(S)-amino-5-cyclohexyl-3(S)-hydroxypentanoic acid isobutylamide |
| ACHPA | 4(S)-amino-5-cyclohexyl-3(S)-hydroxypentanoic acid |
| BOC | tert-butoxycarbonyl |
| CBZ | benzyloxycarbonyl |
| $^{i}$Bu | isobutyl |
| API | 3-(1-imidazolyl)propylamino |

TABLE 1

| $R_s$-ACHPA-$R_t$ | | | |
|---|---|---|---|
| Desc.No. | $R_s$ | $R_t$ | salt/solvate |
| D3(a) | BOC | API | |
| D3(b) | CBZ-Phe-Leu | API | |
| D3(c) | Phe-Leu | API | |
| D4(a) | BOC-Phe-Leu | NH$^{i}$Bu | |
| D4(b) | Phe-Leu | NH$^{i}$Bu | $CF_3CO_2H$ |
| D5(a) | CBZ-Phe-Leu | NH$^{i}$Bu | |
| D5(b) | Phe-Leu | NH$^{i}$Bu | $CH_3CO_2H$ |
| D5(c) | Phe-Leu | NH$^{i}$Bu | |
| D9(a) | BOC-Phe-Leu | API | |
| D9(b) | Phe-Leu | API | $2CF_3CO_2H$ |

TABLE 2

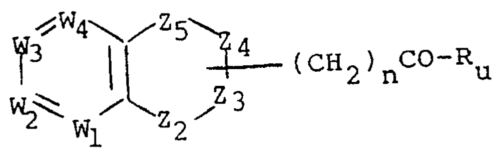

| Desc.No. | $W_1$ | $W_2$ | $W_3$ | $W_4$ | $Z_2$ | $Z_3$ | $Z_4$ | $Z_5$ | n | $R_u$ |
|---|---|---|---|---|---|---|---|---|---|---|
| D1 | CH | CH | CH | N | – | O | $CH_2$ | CH* | 1 | OH |
| D2(a) | CH | CH | CH | N | O | $CH_2$ | CO | N* | 2 | OMe |
| D2(b) | CH | CH | CH | N | O | $CH_2$ | CO | N* | 2 | OH |
| D6(b) | CH | CH | N | CH | S | $CH_2$ | CO | N* | 2 | OMe |
| D6(b) | CH | CH | N | CH | S | $CH_2$ | CO | N* | 2 | OH |
| D7(c) | CH | CH | CH | N | – | NH | CO | N* | 2 | OEt |
| D7(d) | CH | CH | CH | N | – | NH | CO | N* | 2 | OH |
| D8(c) | N | CH | CH | CH | – | NH | CO | N* | 2 | OMe |
| D8(d) | N | CH | CH | CH | – | NH | CO | N* | 2 | OH |
| D10(a) | CH | CH | N | CH | S | $CH_2$ | CO | N* | 2 | $O^tBu$ |
| D10(b) | CH | CH | N | CH | $SO_2$ | $CH_2$ | CO | N* | 2 | $O^tBu$ |
| D10(c) | CH | CH | N | CH | $SO_2$ | $CH_2$ | CO | N* | 2 | OH |
| D11(b) | N | CH | CH | CH | S | $CH_2$ | CO | N* | 2 | $O^tBu$ |
| D11(c) | N | CH | CH | CH | S | $CH_2$ | CO | N* | 2 | OH |
| D12(a) | N | CH | CH | CH | $SO_2$ | $CH_2$ | CO | N* | 2 | $O^tBu$ |
| D12(b) | N | CH | CH | CH | $SO_2$ | $CH_2$ | CO | N* | 2 | OH |
| D13(a) | N | CH | CH | CH | – | CO | N* | CO | 3 | OEt |
| D13(b) | N | CH | CH | CH | – | CH(OH) | N* | CO | 3 | OEt |
| D13(c) | N | CH | CH | CH | – | $CH_2$ | N* | CO | 3 | OEt |
| D13(d) | N | CH | CH | CH | – | $CH_2$ | N* | CO | 3 | OH |

TABLE 2 (Cont'd)

| Desc.No. | $W_1$ | $W_2$ | $W_3$ | $W_4$ | $Z_2$ | $Z_3$ | $Z_4$ | $Z_5$ | n | $R_u$ |
|---|---|---|---|---|---|---|---|---|---|---|
| D14(a) | N | CH | CH | CH | - | CO | N* | CH(OH) | 3 | OEt |
| D14(b) | N | CH | CH | CH | - | CO | N* | $CH_2$ | 3 | OEt |
| D14(c) | N | CH | CH | CH | - | CO | N* | $CH_2$ | 3 | OH |
| D15(a) | CH | N | CH | CH | - | CH | N* | CO | 3 | OEt |
| D15(b) | CH | N | CH | CH | - | CO | N* | $CH_2$ | 3 | OEt |
| D15(c) | CH | N | CH | CH | - | CO | N* | $CH_2$ | 3 | OH |
| D16(a) | CH | CH | CH | N | CH | CH | CO | N* | 3 | OH |
| D16(b) | CH | CH | CH | N | $CH_2$ | $CH_2$ | CO | N* | 3 | OH |
| D17(a) | N | CH | CH | CH | CH | CH | CO | N* | 3 | OH |
| D17(b) | N | CH | CH | CH | $CH_2$ | $CH_2$ | CO | N* | 3 | OH |
| D20(d) | N | CH | CH | CH | O | $CH_2$ | CO | N* | 2 | OH |
| D22(b) | CH | CH | N | CH | O | $CH_2$ | CO | N* | 2 | OH |

TABLE 3

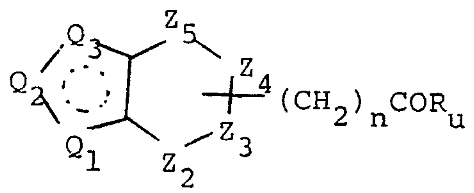

| Desc.No. | $Q_1$ | $Q_2$ | $Q_3$ | $Z_2$ | $Z_3$ | $Z_4$ | $Z_5$ | n | $R_u$ |
|---|---|---|---|---|---|---|---|---|---|
| D18(a) | S | CH | CH | CO | N* | $CH_2$ | $CH_2$ | 4 | OEt |
| D18(b) | S | CH | CH | CO | N* | $CH_2$ | $CH_2$ | 4 | OH |

## TABLE 4

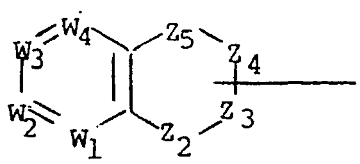

$(CH_2)_n$CO-Phe-Leu-ACHPAR$_t$

| Ex. No. | $W_1$ | $W_2$ | $W_3$ | $W_4$ | $Z_2$ | $Z_3$ | $Z_4$ | $Z_5$ | n | $R_t$ | salt/ solvate |
|---|---|---|---|---|---|---|---|---|---|---|---|
| E1 | CH | CH | CH | N | - | O | $CH_2$ | CH* | 1 | $NH^iBu$ | $0.5H_2O$ |
| E2 | CH | CH | CH | NO | - | O | $CH_2$ | CH* | 1 | $NH^iBu$ | |
| E3 | CH | CH | CH | N | O | $CH_2$ | CO | N* | 2 | API | $0.5CHCl_3$ |
| E4a | CH | CH | N | CH | S | $CH_2$ | CO | N* | 2 | API | $2H_2O$ |
| E4b | CH | CH | N | CH | S | $CH_2$ | CO | N* | 2 | API | HCl |
| E5 | CH | CH | CH | N | - | O | $CH_2$ | CH* | 1 | API | |
| E6 | CH | CH | CH | N | - | NH | CO | N* | 2 | API | HCl |
| E7 | N | CH | CH | CH | - | NH | CO | N* | 2 | API | HCl |
| E8 | CH | CH | CH | NO | - | O | $CH_2$ | CH* | 1 | API | 2HCl |
| E9 | CH | CH | N | CH | $SO_2$ | $CH_2$ | CO | N* | 2 | API | HCl |
| E10 | N | CH | CH | CH | S | $CH_2$ | CO | N* | 2 | API | HCl |
| E11 | N | CH | CH | CH | $SO_2$ | $CH_2$ | CO | N* | 2 | API | $HCl.1.5H_2O$ |
| E12 | N | CH | CH | CH | - | $CH_2$ | N* | CO | 3 | API | HCl |
| E13 | N | CH | CH | CH | - | CO | N* | $CH_2$ | 3 | API | HCl |
| E14 | CH | N | CH | CH | - | CO | N* | $CH_2$ | 3 | API | 2HCl |
| E15 | CH | CH | CH | N | $CH_2$ | $CH_2$ | CO | N* | 3 | API | HCl |
| E16 | N | CH | CH | CH | $CH_2$ | $CH_2$ | CO | N* | 3 | API | HCl |
| E19 | N | CH | CH | CH | O | $CH_2$ | CO | N* | 2 | API | |
| E21 | CH | CH | N | CH | O | $CH_2$ | CO | N* | 2 | API | |

TABLE 5

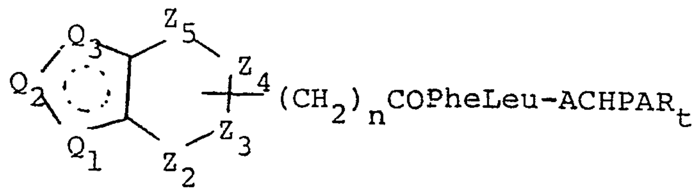

| Ex.No. | $Q_1$ | $Q_2$ | $Q_3$ | $Z_2$ | $Z_3$ | $Z_4$ | $Z_5$ | n | $R_t$ | Salt/ Solvate |
|---|---|---|---|---|---|---|---|---|---|---|
| E17a | S | CH | CH | CO | N* | $CH_2$ | $CH_2$ | 4 | API | $H_2O$ |
| E17b | S | CH | CH | CO | N* | $CH_2$ | $CH_2$ | 4 | API | $HCl.1.5H_2O$ |
| E21 | S | CH | CH | CO | N* | $CH_2$ | $CH_2$ | 3 | API | |

TABLE 6

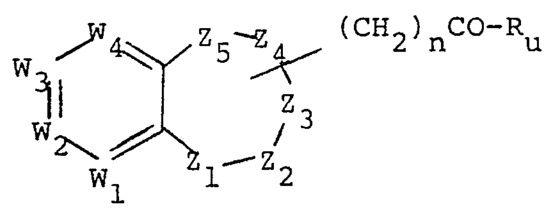

| Desc/ Ex.No. | $W_1$ | $W_2$ | $W_3$ | $W_4$ | $Z_1$ | $Z_2$ | $Z_3$ | $Z_4$ | $Z_5$ | n | $R_u$ |
|---|---|---|---|---|---|---|---|---|---|---|---|
| D19(d) | N | CH | CH | CH | S | $CH_2$ | $CH_2$ | CO | N* | 2 | OMe |
| D19(e) | N | CH | CH | CH | S | $CH_2$ | $CH_2$ | CO | N* | 2 | OH |
| D21(c) | CH | CH | N | CH | S | $CH_2$ | $CH_2$ | CO | N* | 2 | OH |
| E18 | N | CH | CH | CH | S | $CH_2$ | $CH_2$ | CO | N* | 2 | Phe-Leu-ACHPA-API |
| E20 | CH | CH | N | CH | S | $CH_2$ | $CH_2$ | CO | N* | 2 | Phe-Leu-ACHPA-API |

TABLE 7

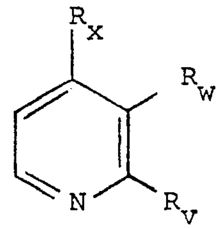

| Desc.No. | $R_V$ | $R_W$ | $R_X$ |
|---|---|---|---|
| D7(a) | $NHCH_2CH_2CO_2Et$ | $NO_2$ | H |
| D7(b) | $NHCH_2CH_2CO_2Et$ | $NH_2$ | H |
| D8(a) | $NHCO_2Et$ | $NHCO_2Et$ | H |
| D8(b) | - NHCONH - | | H |
| D11(a) | - $SCH_2CONH$ - | | H |
| D18(a) | $SCH_2CH_2CO_2H$ | $NO_2$ | H |
| D19(b) | $SCH_2CH_2CO_2H$ | $NH_2$ | H |
| D19(c) | - $SCH_2CH_2CONH$ - | | H |
| D20(a) | $OCH_2CO_2Et$ | $NO_2$ | H |
| D20(b) | $OCH_2CO_2Et$ | $NH_2$ | H |
| D20(c) | - $OCH_2CONH$ - | | H |
| D21(a) | H | $NO_2$ | $SCH_2CH_2CO_2H$ |
| D21(b) | H | - $NHCOCH_2CH_2S$ - | |
| D22(a) | H | - $NHCOCH_2O$ - | |

Description 1

2,3-Dihydrofuro[3,2-b]pyridine-3-acetic acid

10% Aqueous sodium hydroxide (0.7ml) was added dropwise to a stirred solution of ethyl 2,3-dihydrofuro[3,2-b]pyridine-3-acetate * (0.32g) in ethanol (5ml) at 0° C, and the resultant mixture stirred at room temperature for 18h. Water (50ml) was added and the aqueous solution washed with chloroform. The aqueous layer was neutralised with 10% aqueous citric acid and evaporated *in vacuo* . The residue was triturated with chloroform and filtered and the filtrate was evaporated *in vacuo* to afford the title compound (0.17g) as a white solid.

NMR (δ) ($DMSOd_6$): 2.1(1H,dd), 2.6(1H,dd), 3.65(1H,m), 4.25(1H,t), 4.8(1H,t), 7.05(2H,m), 7.95(1H,m)

* Tet Letts 1985, 26, 49, 6001-6004.

Description 2

(a) Methyl 2,3-dihydro-3-oxo-4H-pyrido[3,2-b]-1,4-oxazine-4-propanoate

Methyl acrylate (0.18ml) was added dropwise to a stirred mixture of 2,3-dihydro-3-oxo-4H-pyrido[3,2-b]-1,4-oxazine (0.50g) and anhydrous potassium carbonate (0.41g) in dry dimethylformamide (DMF) (5ml). The reaction mixture was stirred at room temperature for 66h, poured into water and extracted with chloroform (3x100ml). Combined extracts were dried ($Na_2SO_4$) and the solvent removed in vacuo . The crude product was purified by chromatography on silica gel using ethyl acetate/petroleum ether (b.p. 60-80 ° C) (0-30% ethyl acetate, gradient) to afford the title compound (0.48g) as a white solid.
NMR (δ) ($CDCl_3$): 2.75(2H,t), 3.65(3H,s), 4.45(2H,t), 4.7(2H,s), 6.9(1H,dd), 7.2(1H,dd), 8.0(1H,dd).

(b) 2,3-Dihydro-3-oxo-4H-pyrido[3,2-b1-1,4-oxazine-4-prooanoic acid

10% Aqueous sodium hydroxide (0.56ml) was added to a chilled, stirred solution of methyl 2,3-dihydro-3-oxo-4 H -pyrido[3,2-b]-1,4-oxazine-4-propanoate (0.47g) in methanol (5ml) and the resultant mixture stirred at room temperature for 21h. Solvents were evaporated in vacuo and the residue dissolved in water and adjusted to neutrality with 2M hydrochloric acid. The solution was freeze-dried and the residue triturated with methanol/dichloromethane (1:5 mixture). Filtration and evaporation of the filtrate afforded the title compound (0.40g) as a white solid.
NMR (δ) ($DMSOd_6$): 2.2(2H,t), 4.1(2H,t), 4.7(2H,s), 7.0(1H,m) 7.35(1H, dd), 8.0(1H,dd).

Description 3

(a) BOC-ACHPA 3-(1-imidazolyl)propylamide

To a stirred ice cooled mixture of BOC-ACHPA-OH (1.2g) and 1-hydroxybenzotriazole (HOBT) (0.51g) in dry DMF (10 ml) was added 1-(3-dimethylaminopropyl)-3-ethylcarbodiimide.HCl (DEC) (0.73g). After 10 min N-(3-aminopropyl)imidazole (0.68ml) was added and stirring continued for 16h. Water was added and the solution extracted with ethyl acetate (3x200ml). The combined extracts were washed with water, saturated sodium hydrogen carbonate and brine, dried ($Na_2SO_4$) and the solvent evaporated in vacuo . The residue was purified by chromatography on silica gel using methanol/chloroform (0-3% methanol, gradient) to afford the title compound (1.45g).
NMR (δ) ($DMSOd_6$): 0.7-1.9(24H,m), 2.15(2H,m), 3.0(2H,m), 3.8(1H,m), 3.95(2H,t), 4.7(1H,m), 6.25(1H,d), 7.1-(1H,s), 7.6(1H,s) 7.8(1H,t).

(b) CBZ-Phe-Leu-ACHPA 3-(1-imidazolyl)propylamide

Chilled trifluoracetic acid (TFA) (20ml) was added to a stirred solution of BOC-ACHPA 3-(1-imidazolyl)-propylamide (1.4g) in dry dichloromethane (20ml) and the reaction mixture stirred at room temperature for lh. Evaporation in vacuo afforded a light yellow foam (1-8g). This material was dissolved in dry DMF (10 ml) and triethylamine (1.37ml) and added to a stirred mixture of CBZ-Phe-Leu-OH (2.02g), HOBT.(0.66g) and DEC (0.94g) in DMF (20ml) at 0 ° C. After 16h water was added and the mixture extracted with ethyl acetate (3x250ml). Combined extracts were washed with water, saturated sodium hydrogen carbonate and brine, dried ($Na_2SO_4$) and evaporated in vacuo . The crude product was purified by chromatography on silica gel using methanol/chloroform (0-5% methanol, gradient) to afford the title compound (1.95g).
NMR (δ) ($CDCl_3$) 0.7-2.5(26H,m), 2.8-3.4(6H,m), 3.9-4.5(6H,m), 5.0(2H,m), 6.6(1H,m), 6.9-7.4(13H,m).
M.S. (m/z) (FAB) (M+1) = 717 (consistent with m.w. = 716)

(c) Phe-Leu-ACHPA 3-(1-imidazolyl)propylamide

CBZ-Phe-Leu-ACHPA 3-(1-imidazolyl)propylamide (1.8g) in ethanol (150ml) was hydrogenated at room

temperature and pressure in the presence of 10% palladium on carbon for 20h. Filtration through kieselguhr and evaporation of the filtrate afforded the crude product which was purified by chromatography on silica gel using methanol/chloroform (0-12% methanol, gradient) to afford the title compound (0.31g).
NMR (δ) ($CD_3OD$): 0.8-1.7(22H,m), 2.0(2H,m), 2.25(2H,m), 2.8(1H,dd) 3.0-3.2(3H,m), 3.65(1H,m), 3.9-4.2-(4H,m), 4.3(1H,m), 6.9(1H,s), 7.15(1H,s), 7.25(5H,m), 7.7(1H,s).

Description 4

(a) BOC-Phe-Leu-ACHPA isobutylamide

This material was formed from BOC-Phe-Leu-OH (1.48g) and ACHPA isobutylamide * (1.06g), following the procedure of Description 3(a). The crude product was chromatographed on silica gel using methanol/chloroform (0-2% methanol, gradient). This gave the title compound (1.91g).
NMR (δ) ($CDCl_3$): 0.7-2.3 (31 H,m), 2.8-3.2(4H,m), 3.8-4.3(4H,m), 6.4-7.4(8H,m).

(b) Phe-Leu-ACHPA isobutylamide.TFA

BOC-Phe-Leu-ACHPA isobutylamide (2.32g) was dissolved with stirring in TFA (10ml) at 0 °C. After 1h, solvent was removed in vacuo , and the residue was triturated with ether to give the title compound.
NMR (δ) ($CDCl_3$): 0.6-2.5 (31H,m), 2.8-3.3(4H,m), 3.7-4.4(4H,m), 7.0-8.0(8H,m).

Description 5

(a) CBZ-Phe-Leu-ACHPA isobutylamide

This material was formed from CBZ-Phe-Leu-OH (4.13g), following the procedure of Description 4(a). The crude product was purified by chromatography on silica gel using methanol/chloroform (0-3% methanol, gradient). This gave the title compound (6.40g) as a light brown solid.
NMR (δ) ($CDCl_3$): 0.75-1.85 (29H,m), 2.3(2H,m), 2.9-3.15 (4H,m), 4.0(2H,m), 4.3-4.5(2H,m), 5.05(2H,s), 5.5-(1H,d), 6.5-6.85(3H,m), 7.1-7.4(11H,m).

(b) Phe-Leu-ACHPA isobutylamide.$CH_3CO_2H$

This material was formed from CBZ-Phe-Leu-ACHPA isobutylamide (6.4g) following the procedure of Description 3(c), but using ethanol/acetic acid as solvent. This gave the title compound (7.4g), still containing residual acetic acid.
NMR (δ) ($CDCl_3$): 0.8-1.85(29H,m), 2.05(m), 2.35(2H,m), 2.9-3.2(4H,m), 3.95(2H,m), 4.15(1H,m), 4.3(1H,m), 7.25(5H,m), 8.0(b).

(c) Phe-Leu-ACHPA isobutylamide

Phe-Leu-ACHPA isobutylamide.$CH_3CO_2H$ (7.4g) was dissolved in ethyl acetate and extracted into aqueous citric acid. The extract was basified with sodium carbonate, and the product was isolated by extraction into chloroform, drying ($Na_2SO_4$) and evaporation in vacuo . This gave the title compound (3.2g).
NMR (δ) ($CDCl_3$): 0.7-1.0 (13H,m), 1.0-1.9(16H,m), 2.3(2H,ABX), 2.75(1H,dd), 3.05(2H,m), 3.2(1H,dd), 3.65-(1H,dd), 3.95(2H,m), 4.35(1H,m), 6.5(1H,d), 6.6(1H,t), 7.15-7.35(6H,m), 7.7(1H,d).

Description 6

* J. Med. Chem. 1985, 28 , 1779-1790.

a) Methyl 2,3-dihydro-3-oxo-4H-pyrido[4,3-b]-1,4-thiazine-4-propanoate

This material was prepared from 2,3-dihydro-3-oxo-4 H -pyrido[4,3-b]-1,4-thiazine * (0.18g), following the procedure of Description 2(a). Purification by chromatography on silica gel using methanol/chloroform (0-4% methanol, gradient) afforded the title compound (0.18g) as a colourless waxy solid.
NMR (δ) ($CDCl_3$): 2.75 (2H,t), 3.45 (2H,s), 3.7 (3H,s), 4.35 (2H,t), 7.3 (1H,dd), 8.2 (1H,d), 8.45 (1H,s).

b) 2,3-Dihydro-3-oxo-4H-pyrido[4,3-b]-1,4-thiazine-4-propanoic acid

This material was prepared from methyl 2,3-dihydro-3-oxo-4 H -pyrido[4,3-b]-1,4-thiazine-4-propanoate (0.17g), following the procedure of Description 2(b). This gave the title compound (0.15g) as a white solid.
NMR (δ) ($DMSOd_6$): 2.15 (2H,m), 3.55 (2H,s), 4.1 (2H,m), 7.4 (1H,d), 8.1 (1H,d), 8.5 (1H,s).

Description 7

a) Ethyl 3-((3-nitro-2-pyridyl)amino)propanoate

2-Chloro-3-nitropyridine (2.0g), ethyl 3-amino-propanoate.HCl (1.94g) and anhydrous potassium carbonate (3.48g) were stirred in DMF (20 ml) for 16h. The mixture was evaporated in vacuo , partitioned between chloroform and water, and separated. The aqueous portion was extracted with chloroform, and the combined organics were washed with water and brine, dried ($Na_2SO_4$) and evaporated in vacuo . Chromatography on silica gel using methanol/chloroform (0-2% methanol, gradient) gave the title compound (2.5g).
NMR (δ) ($CDCl_3$): 1.3 (3H,t), 2.7 (2H,t), 3.95 (2H,q), 4.2 (2H,q), 6.7 (1H,dd), 8.4-9.7 (2H,m).

b) Ethyl 3-((3-amino-2-pyridyl)amino)propanoate

This material was formed from ethyl 3-((3-nitro-2-pyridyl)amino)propanoate (2.90g), following the procedure of Description 3(c). This gave the title compound (2.43g) as a red oil.
NMR (δ) ($CDCl_3$): 1.3 (3H,t), 2.7 (2H,t), 3.2 (2H,b), 3.75 (2H,m), 4.15 (2H,q), 4.8 (1H,b), 6.5 (1H,dd), 6.85 (1H,d), 7.7 (1H,d).

c) Ethyl 2,3-dihydro-2-oxo-1H-imidazo[4,5-b]-e-3-propanoate

Ethyl 3-((3-amino-2-pyridyl)amino)propanoate (2.4g) was stirred in dichloromethane (50 ml) as 1,1′-carbonyldiimidazole (2.27g) was added. After a vigorous reaction, the mixture was stirred for 16h, evaporated in vacuo . dissolved in ethyl acetate, washed with water, dried ($Na_2SO_4$) and evaporated in vacuo . Chromatography on silica gel using methanol/chloroform (0-5% methanol, gradient) gave the title compound (2.22g) as a purple solid.
NMR (δ) ($CDCl_3$): 1.2 (3H,t), 2.9 (2H,t), 4.15 (2H,q), 4.35 (2H,t), 5.7 (b), 7.0 (1H,dd), 7.3 (1H,dd), 8.05 (1H,dd), 10.4 (b).

d) 2,3-Dihydro-2-oxo-1H-imidazo[4,5-b]pyridin-3-propanoic acid

This material was formed from ethyl 2,3-dihydro-2-oxo-1 H -imidazo[4,5-b]pyridine-3-propanoate (0.82g), following the procedure of Description 2(b), but using ethanol as solvent. This gave the title compound (0.8g) as a pink solid.
NMR (δ) ($DMSOd_6$): 2.3 (2H,t), 3.95 (2H,t), 6.9 (1H,dd), 7.25 (1H,d), 7.85 (1H,d).

* Synth. Comm. 331-3 (1985).

Description 8

a) 2,3-Di(ethoxycarbonylamino)pyridine

2,3-Diaminopyridine (2.77g) and dry pyridine (8.1 ml) were stirred at 0° C in dry tetrahydrofuran (THF) (50 ml) as ethyl chloroformate (4.9 ml) was added dropwise. The mixture was stirred at 0° C for 15 min, and then allowed to rise to ambient temperature over 3h. It was diluted with ethyl acetate, washed with water, dried ($Na_2SO_4$) and evaporated in vacuo . Chromatography on alumina using 10% ethyl acetate in petroleum ether (b.p. 60-80° C) gave the title compound (3.16g) as an orange solid.
NMR (δ) ($CDCl_3$): 1.35 (6H, 2xt), 4.25 (4H, 2xq), 7.2 (1H,dd), 8.1 (1H,d), 8.2 (1H,d), 8.45 (1H,b), 8.8 (b).

b) 2,3-Dihydro-2-oxo-1H-imidazo[4,5-b]pyridine

2,3-Di(ethoxycarbonylamino)pyridine (3.16g) was dissolved in 2M aqueous sodium hydroxide (100 ml) at 50° C. The solution was then acidified (5M hydrochloric acid) and neutralised with sodium hydrogen carbonate, and extracted with ethyl acetate. The extract was dried ($Na_2SO_4$) and evaporated in vacuo to give the title compound (0.20g), as a brown solid. On standing, the aqueous portion yielded a brown precipitate, which was filtered off and dried in vacuo , affording a further crop of the title compound (1.14g).
NMR (δ) ($DMSOd_6$): 6.9 (1H,dd), 7.2 (1H,d), 7.8 (1H,d), 11.1 (1H,vb).

c) Methyl 2,3-dihydro-2-oxo-1H-imidazo[4,5-b]-pyridine-1-propanoate

This material was formed from 2,3-dihydro-2-oxo-1-1 H -imidazo[4,5-b]pyridine (1.3g), following the procedure of Description 2(a). The crude product was purified by chromatography using ethyl acetate/petroleum ether (b.p. 60-80° C) (0-100% ethyl acetate, gradient). This gave the title compound (1.2g).
NMR (δ) ($CDCl_3$): 2.85 (2H,t), 3.65 (3H,s), 4.2 (2H,t), 7.05 (1H,dd), 7.4 (1H,d), 8.1 (1H,d).

d) 2,3-Dihydro-2-oxo-1H-imidazo[4.5-b]pyridine-1-propanoic acid

This material was formed from methyl 2,3-dihydro-2-oxo-1 H -imidazo[4,5-b]pyridine-1-propanoate (0.78g), following the procedure of Description 2(b). This gave the title compound (0.42g) as a white solid.
NMR (δ) ($DMSOd_6$): 2.25 (2H,t), 3.95 (2H,t), 6.9 (1H,dd), 7.4 (1H,d), 7.8 (1H,d).

Description 9

a) BOC-Phe-Leu-ACHPA 3-(1-imidazolyl)propylamide

This material was formed from BOC-ACHPA (3-(1-imidazolyl)propylamide (1.53g) (Description 3(a)) and BOC-Phe-Leu-OH (1.60g), following the procedure of Description 3(b). The crude product was chromatographed on silica gel using methanol/chloroform (0-10% methanol, gradient). This gave the title compound (1.95g).
NMR (δ) ($CDCl_3$): 0.7-1.05 (8H,m), 1.05-1.9 (2H,m), 2.0 (2H,m), 2.2-2.5 (2H,m), 2.9-3.6 (5H,m), 3.9-4.5 (6H,m), 5.1 (b), 6.4 (b), 6.6 (bd), 7.0 (1H,s), 7.05 (1H,s), 7.15-7.4 (m), 7.65 (1H,s).

b) Phe-Leu-ACHPA 3-(1-imidazolyl)propylamide .2($CF_3CO_2H$)

To a solution of BOC-Phe-Leu-ACHPA 3-(1-imidazolyl)propylamide (0.60g) in dichloromethane (6 ml) was added trifluoroacetic acid (6 ml). The mixture was stirred for 1h, and evaporated. Trituration with ether/pentane and filtration then gave the title compound (0.62g) as a white solid.

Description 10

a) t-Butyl 2,3-dihydro-3-oxo-4H-pyrido[4,3-b]-1,4-thiazine-4-propanoate

This material was formed from 2,3-dihydro-3-oxo-4 H -pyrido[4,3-b]-1,4-thiazine (0.36g) and t-butyl acrylate (0.63 ml), following the procedure of Description 2(a). This gave the title compound (0.62g) as a pale orange oil.
NMR (δ) ($CDCl_3$): 1.4 (9H,s), 2.65 (2H,t), 3.45 (2H,s), 4.3 (2H,t), 7.3 (1H,d), 8.2 (1H,d), 8.45 (1H,s).

b) t-Butyl 2,3-dihydro-1,1,3-trioxo-4H-pyrido[4,3-b]-1,4-thiazine-4-propanoate

t-Butyl 2,3-dihydro-3-oxo-4H-pyrido[4,3-b]-1,4-thiazine-4-propanoate (0.18g) and monoperoxyphthalic acid, magnesium salt .$6H_2O$ (0.60g) were stirred in methanol (5 ml) for 16h. The mixture was evaporated in vacuo , taken up in ether, washed with 5% sodium hydrogen carbonate solution and brine, dried ($Na_2SO_4$) and evaporated in vacuo . The residue was taken up in chloroform, filtered and evaporated in vacuo , giving the title compound (0.092g) as a colourless oil.
NMR (δ) ($CDCl_3$): 1.4 (9H,s), 2.7 (2H,t), 4.3 (2H,s), 4.4 (2H,t), 7.8 (1H,d), 8.7 (1H,d), 8.9 (1H,s).

c) 2,3-Dihydro-1,1,3-trioxo-4H-pyrido[4,3-b]-1,4-thiazine-4-propanoic acid

t-Butyl 2,3-dihydro-1,1,3-trioxo-4 H -pyrido[4,3-b]-1,4-thiazine-4-propanoate (0.15g) was dissolved in dichloromethane (2 ml) and TFA (2 ml), and left to stand for 3h. Solvent was evaporated in vacuo , and the residue was triturated with $Et_2O$. Filtration then gave the title compound (0.063g) as a white powder.
NMR (δ) ($DMSOd_6$): 2.6 (2H,t), 4.3 (2H,t), 4.95 (2H,s), 7.85 (1H,d), 8.65 (1H,d), 9.05 (1H,s).

Description 11

a) 2,3-Dihydro-3-oxo-4H-pyrido[2,3-b]-1,4-thiazine

2-Chloro-3-nitropyridine (2.31g), anhydrous potassium carbonate (4.02g) and ethyl mercaptoacetate (2.4 ml) was stirred in DMF (30 ml) for 1.5h. The mixture was diluted with ethyl acetate (300 ml), washed with water and brine, dried ($Na_2SO_4$) and evaporated in vacuo to give a dark green mobile oil (4.53g). This was dissolved in glacial acetic acid (100 ml), and stirred as iron powder (20g) was added. The mixture was stirred at 90° C for 2h, cooled, and evaporated in vacuo . The resultant brown solid was extracted twice with xylene, and the extracts were evaporated to give the title compound (0.85g), impure, as a brown solid. The residue remaining after xylene extraction was dissolved in 10% aq. acetic acid, filtered, and extracted with 10% methanol/chloroform. This extract was dried ($Na_2SO_4$) and evaporated in vacuo , giving a second crop of material (0.88g), pure, as a buff solid. The initial xylene extract was purified by trituration with ether, giving a total yield of 1.38g.
NMR (δ) ($DMSOd_6$): 3.65 (2H,s), 7.2 (2H,m), 8.1 (1H,dd), 10.65 (1H,b).

b) t-Butyl 2,3-dihydro-3-oxo-4H-pyrido[2,3-b]-1,4-thiazine-4-propanoate

This material was formed from 2,3-dihydro-3-oxo-4 H -pyrido[2,3-b]-1,4-thiazine (0.84g), following the procedure of Description 10(a). This gave the title compound (1.57g) as a brown oil.
NMR (δ) ($CDCl_3$): 1.4 (9H,s), 2.6 (2H,t), 3.5 (2H,s), 4.2 (2H,t), 7.2 (1H,dd), 7.5 (1H,dd), 8.2 (1H,dd).

c) 2,3-Dihydro-3-oxo-4H-pyrido[2,3-b]-1,4-thiazine-4-propanoic acid

This material was formed from t-butyl 2,3-dihydro-3-oxo-4 H -pyrido[2,3-b]-1,4-thiazine-4-propanoate (0.33g), following the procedure of Description 10(c). This gave the title compound (0.24g) as a white

powder.
NMR (δ) ($CDCl_3$/$DMSOd_6$): 2.55 (m), 3.5 (s), 4.15 (2H,t), 7.25 (1H,dd), 7.65 (1H,d), 8.1 (1H,dd).

Description 12

a) t-Butyl 2,3-dihydro-1,1,3-trioxo-4H-pyrido[2,3-b]-1,4-thiazine-4-propanoate

This material was formed from t-butyl 2,3-dihydro-3-oxo-4 H -pyrido[2,3-b]-1,4-thiazine-4-propanoate (Description 11(b)) (0.40g), following the procedure of Description 10(b). This gave the title compound (0.29g) as a clear oil, but contaminated with ca.20% of an unidentified impurity (NMR).
NMR (δ) ($CDCl_3$): 1.4 (9H,s), 2.65 (2H,t), 4.3 (2H,t), 4.35 (2H,s), 7.65 (1H,dd), 7.9 (1H,d), 8.55 (1H,dd).

b) 2,3-Dihydro-1,1,3-trioxo-4H-pyrido[2,3-b]-1,4-thiazine-4-propanoic acid

This material was formed from t-butyl 2,3-dihydro-1,1,3-trioxo-4 H -pyrido[2,3-b]-1,4-thiazine-4-propanoate (0.26g), following the procedure of Description 10(c). This gave the pure title compound (0.16g) as a grey solid.
NMR (δ) ($DMSOd_6$): 2.55 (2H,t), 4.2 (2H,t), 4.95 (2H,s), 7.8 (1H,dd), 8.2 (1H,d), 8.5 (1H,d).

Description 13

a) Ethyl 5,7(6H)-dioxo-5H-pyrrolo[3,4-b]pyridine-6-butanoate

A mixture of pyridine-2,3-dicarboxylic acid (15g) and urea (6.65g) was heated at 180°C for 30 min. After cooling to room temperature, the residue was triturated with ethyl acetate and water to give the crude dicarboximide intermediate.

This crude intermediate was alkylated with ethyl 4-bromobutyrate following the procedure of Description 2(a) to give the title compound (3.75g).
NMR (δ) ($CDCl_3$): 1.3 (3H,t), 2.0 (2H,quin), 2.4 (2H,t), 3.8 (2H,t), 4.1 (2H,q), 7.6 (1H,dd), 8.1 (1H,d), 9.0 (1H,d).

b) Ethyl 6,7-dihydro-7-hydroxy-5-oxo-5H-pyrrolo-[3,4-b]pyridine-6-butanoate

To a stirred solution of ethyl 5,7(6H)-dioxo-5 H -pyrrolo[3,4-b]pyridine-6-butanoate (0.95g) in methanol (55ml) at 0°C was added sodium borohydride (0.28g). After 20 min, the reaction was quenched by the addition of 5M hydrochloric acid, and the reaction mixture evaporated. The residue was partitioned between chloroform (50 ml) and aqueous sodium hydrogen carbonate (20 ml), then the organic phase was dried and evaporated. Chromatography on silica using ethyl acetate gave the title compound (0.68g).
NMR (δ) ($CDCl_3$): 1.2 (3H,t), 2.0-2.2 (2H,m), 2.4-2.5 (2H,m), 3.6-3.7 (2H,m), 4.1 (2H,q), 7.5 (1H,dd), 8.1 (1H,d), 8.8 (1H,d).

c) Ethyl 6,7-dihydro-5-oxo-5H-pyrrolo[3,4-b]-pyridine-6-butanoate

A solution of ethyl 6,7-dihydro-7-hydroxy-5-oxo-5 H -pyrrolo[3,4-b]pyridine-6-butanoate (0.33g) in acetic acid (8 ml) was maintained under reflux for 13h in the presence of zinc dust (0.42g). The reaction mixture was filtered and evaporated, then the title compound (0.24g) was obtained by chromatography on silica using ethyl acetate.
NMR (δ) ($CDCl_3$): 1.2 (3H,t), 2.0 (2H, quin), 2.4 (2H,t), 3.7 (2H,t), 4.0 (2H,q), 4.8 (2H,s), 7.7 (1H,t), 8.4 (1H,d), 8.9 (1H,bs).

d) 6,7-Dihydro-5-oxo-5H-pyrrolo[3,4-]pyridine-6-butanoic acid

To a solution of ethyl 6,7-dihydro-5-oxo-5H-pyrrolo[3,4-b]pyridine-6-butanoate (0.36g) in ethanol (7 ml) was added water (7 ml) and potassium carbonate (0.20g). This mixture was stirred at room temperature for 3 days, then evaporated. Chromatography on silica using 50% methanol/chloroform gave the title compound (0.18g).
NMR (δ) ($DMSOd_6$): 1.8-1.9 (2H,quin), 2.25 (2H,t), 3.5 (2H,t), 4.5 (2H,s), 7.5 (1H,m), 8.1 (d), 8.7 (1H,d).

Description 14

a) Ethyl 6,7-dihydro-5-hydroxy-7-oxo-5H-pyrrolo-[3,4-b]pyridine-6-butanoate

The title compound (0.23g) was obtained as a more slowly eluting by-product of the synthesis of ethyl 6,7-dihydro-7-hydroxy-5-oxo-5 H -pyrrolo[3,4-b]pyridine-6-butanoate (Description 13(b)).
NMR (δ) ($CDCl_3$): 1.3 (3H,t), 1.9-2.1 (2H,m), 2.4-2.5 (2H,m), 3.5-3.8(2H,m), 4.0 (2H,q), 7.4-7.5 (1H,m), 8.0 (1H,d), 8.7 (1H,d).

b) Ethyl 6,7-dihydro-7-oxo-5H-pyrrolo[3,4-b]-pyridine-6-butanoate

A solution of ethyl 6,7-dihydro-5-hydroxy-7-oxo-5 H -pyrrolo[3,4-b]pyridine-6-butanoate (0.23g) in ethanol (25 ml) was acidified with 5M hydrochloric acid, then hydrogenated in the presence of 10% Pd/C for 4 days. The mixture was filtered and the filtrate evaporated to give the title compound (0.17g).
NMR (δ) ($DMSOd_6$): 1.1-1.2 (3H,m), 1.8-1.9 (2H,m), 2.3 (2H,t), 3.6 (2H,t), 4.0 (2H,q), 4.5 (2H,s), 7.6 (1H,dd), 8.1 (1H,d), 8.7 (1H,d).

c) 6,7-Dihydro-7-oxo-5H-pyrrolo[3,4-b]pyridine-6-butanoic acid

This material was formed from ethyl 6,7-dihydro-7-oxo-5 H -pyrrolo[3,4-b]pyridine-6-butanoate (0.17g), following the procedure of Description 13(d). This gave the title compond (0.12g).
NMR (δ) ($DMSOd_6$): 1.8-1.9 (2H,m), 2.2-2.3 (2H,m), 3.6 (2H,t), 4.5 (2H,s), 7.6 (1H,dd), 8.1 (1H,d), 8.7(1H,d).

Description 15

a) Ethyl 5,7(6H)-dioxo-5H-pyrrolo[3,4-c]pyridine-6-butanoate

This material was formed from pyridine-3,4-dicarboximide (2.5g) and ethyl 4-bromobutyrate (2.0ml), following the procedure of Description 2(a). This gave the title compound (3.13g).
NMR (δ) ($CDCl_3$): 1.25 (3H,t), 2.0 (2H, quin), 2.4 (2H,t), 3.8 (2H,t), 4.1 (2H,q), 7.8 (1H,d), 9.05 (1H,d), 9.15 (1H,s).

b) Ethyl 6,7-dihydro-5H-7-oxopyrrolo[3,4-c]-pyridine-6-butanoate

This material was formed from ethyl 5,7(6H)-dioxo-5 H -pyrrolo[3,4-c]pyridine-6-butanoate (0.90g), following the procedures of Descriptions 13(b) and 13(c), but omitting chromatography of the 5-hydroxy intermediate. Chromatography on silica using ethyl acetate gave the title compound (0.41g).
NMR (δ) ($CDCl_3$): 1.2 (3H,t), 2.0 (2H, quin), 2.4 (2H,t), 3.7 (2H,t), 4.05 (2H,q), 4.45 (2H,s), 7.45 (1H,d), 8.75 (1H,d), 9.1 (1H,s).

c) 6,7-Dihydro-5H-7-oxopyrrolo[3,4-c]pyridine-6-butanoic acid.HCl

This material was formed from ethyl 6,7-dihydro-5 H -7- oxopyrrolo[3,4-c]pyridine-6-butanoate (0.30g), following the procedure of Description 13(d), followed by acidification/freeze-drying by the procedure of Example 4(b). This gave the title compound (0.20g).
NMR (δ) (DMSOd$_6$): 1.8 (2H, quin), 2.25 (2H,t), 3.55 (2H,t), 4.7(2H,s), 5.0-6.5 (3H,b), 8.1 (1H,d), 8.9 (1H,d), 9.15 (1H,s).

Description 16

a) 7,8-Dihydro-7-oxo-8H-1,8-naphthyridine-8-butanoic acid

A mixture of 1,8-naphthyridine * (2.5g) and ethyl 4-bromobutyrate (13.7 ml) in ethanol (100 ml) was stirred at reflux for 6 days, then evaporated in vacuo . Water (50 ml) was added and the mixture stirred in an ice bath as a solution of potassium ferricyanide (38g) in water (125 ml) was added dropwise over 0.5h, followed by the dropwise addition of a solution of potassium hydroxide (17.5g) in water (25 ml). Stirring was continued for 24h, during which time the reaction mixture was allowed to warm to room temperature. Water was added to dissolve the remaining solid and the solution washed with chloroform. The aqueous layer was acidified with 5M hydrochloric acid and extracted with chloroform. Pooled chloroform extracts were dried ($Na_2SO_4$) and evaporated in vacuo . The residue was recrystallised from methanol to afford the title compound (2.0g).
NMR (δ) (DMSOd$_6$): 1.9 (2H,m), 2.2 (2H,m), 4.4 (2H,m), 6.7 (1H,d), 7.3 (1H,dd), 7.9(1H,d), 8.2 (1H,dd), 8.6 (1H,m), 12.05 (1H,b).

b) 7-Oxo-5,6,7,8-tetrahydro-8H-1,8-naphthyridine-8-butanoic acid

To stirred palladium black (0.2g) under nitrogen was added 5% formic acid/methanol (8 ml), followed by a solution of 7,8-dihydro-7-oxo-8 H -1,8-naphthyridine-8-butanoic acid (0.40g) in 5% formic acid/methanol (24 ml) in two portions, the latter after 1h reaction. The mixture was stirred at room temperature overnight, filtered through celite and the filtrate evaporated in vacuo . The residue was purified by chromatography on silica gel using methanol/chloroform (0-20% methanol, gradient) to afford the title compound (0.06g).
NMR (δ) ($CDCl_3$): 2.0 (2H,m), 2.4 (2H,t), 2.7 (2H,t), 2.9 (2H,t), 4.2 (2H,t), 6.95 (1H,m), 7.45 (1H,d), 8.2 (1H,d), 9.8 (1H,b).

Description 17

a) 5,6-Dihydro-6-oxo-5H-1,5-naphthyridine-5-butanoic acid

This material was formed from 1,5-naphthyridine * (1.00g) following the procedure of Description 16(a). On evaporation of the chloroform extracts, the solid produced was filtered from the-residual liquid to afford the title compound (0.12g).
NMR (δ) (DMSOd$_6$): 1.8 (2H,m), 2.35 (2H,t), 4.2 (2H,t), 6.9 (1H,dd), 7.6 (1H,dd), 7.9 (1H,dd), 8.1 (1H,d), 9.45 (1H,d), 12.1 (1H,b).

b) 6-Oxo-5,6,7,8-tetrahydro-5H-1,5-naphthyridine-5-butanoic acid

This material was formed from 5,6-dihydro-6-oxo-5 H -1,5-naphthyridine-5-butanoic acid (0.12g), following the procedure of Description 16(b). Isolation by filtration through celite and evaporation of the filtrate in vacuo afforded the title compound (0.12g) which was used without further purification.
NMR (δ) ($CD_3OD$): 1.9 (2H,m), 2.4 (2H,t), 2.75 (2H,t), 3.1 (2H,t), 4.0 (2H,t), 7.35 (1H,dd), 7.7 (1H,d), 8.1 (1H,d).

* Chem. Pharm. Bull. 19 (9) 1857-62 (1971).

Description 18

a) Ethyl 7-oxo-4,5,6,7-tetrahydro-6H-thieno[2,3-c]-pyridine-6-pentanoate

This material was formed from 4,5-dihydrothieno[2,3-c]-pyrid-7(6 H )-one * (0.33g) and ethyl 5-bromovalerate (0.34 ml) following the procedure of Descriptlon 2(a). The crude product was purified by chromatography on silica gel using ethyl acetate/petroleum ether (b.p. 60-80°C) (0-30% ethyl acetate, gradient) to give the title compound (0.35g).
NMR (δ) ($CDCl_3$): 1.2 (3H,t), 1.7 (3H,m), 2.3 (2H,m), 2.9 (2H,t), 3.4-3.7 (4H,m), 4.1 (2H,q), 6.9 (1H,d), 7.4 (1H,d).

b) 7-Oxo-4,5,6,7-tetrahydro-6H-thieno[2,3-c]-pyridine-6-pentanoic acid

This material was formed from ethyl 7-oxo-4,5,6,7-tetrahydro-6 H -thieno[2,3-c]pyridine-6-pentanoate (0.32g) following the procedure of Description 7(d). The title compound (0.27g) was isolated as a slightly coloured oil.
NMR (δ) ($DMSOd_6$): 1.5.(3H,m), 2.2 (2H,m), 2.9 (2H,t), 3.4-3.5 (3H,m), 3.6 (3H,t), 7.0 (1H,d), 7.7 (1H,d).

Description 19

a) 3-((3-Nitro-2-pyridyl)thio)propanoic acid

3-Mercaptopropionic acid (5.0 ml), 2-chloro-3-nitropyridine (9.11g) and anhyd. potassium carbanate (19.8g) were stirred in DMF (100 ml) for 5.5h. Solids were then dissolved by addition of water (200 ml), and the mixture was stirred for 16h, diluted with water (500 ml), washed with ethyl acetate, and acidified with conc. hydrochloric acid. The mixture was then extracted into ethyl acetate, and the extract was dried ($Na_2SO_4$) and evaporated in vacuo , giving the title compound (13.10g) as a yellow solid.
NMR (δ) ($CDCl_3$/$DMSOd_6$): 2.7 (2H,t), 3.4 (2H,t), 7.3 (1H,dd), 8.55 (1H,dd), 8.8 (1H,m).

b) 3-((3-Amino-2-pyridyl)thio)propanoic acid

3-((3-Nitro-2-pyridyl)thio)propanoic acid (9.28g) was hydrogenated in ethanol (200 ml) over 10% palladium on charcoal (60% aq.paste, 4.0g) for 24h. A further 4.1g catalyst was then added, and hydrogenation continued for a further 64h. Filtration through kieselguhr, and evaporation in vacuo then gave the title compound (7.54g) as an orange-yellow solid.
NMR (δ) ($DMSOd_6$): 2.6 (2H,t), 3.3 (2H,t), 5.0 (2H,b), 6.9 (2H,m), 7.75 (1H,m).

c) 3,4-Dihydro-4-oxo-2H-pyrido[2,3-b]-1,4-thiazepine

3-((3-Amino-2-pyridyl)thio)propanoic acid (7.54g) and 1,3-dicyclohexylcarbodiimide (7.84g) were stirred together in DMF (75 ml) for 4.5h, evaporated in vacuo , taken up in ethyl acetate, filtered, and evaporated to a yellow/brown solid. This was purified by chromatography on silica gel using ethyl acetate/petroleum ether (b.p. 60-80°C) (50-100% ethyl acetate, gradient), giving the title compound (1.00g).
NMR (δ) ($CDCl_3$): 2.7 (2H,t), 3.6 (2H,t), 7.3 (1H,dd), 7.4 (1H,dd), 8.05 (1H,b), 8.4 (1H,dd).

d) Methyl 3,4-dihydro-4-oxo-2H-pyrido[2,3-b]-1,4-thiazeoine-5-propanoate

This material was formed from 3,4-dihydro-4-oxo-2 H -pyrido[2,3-b]-1,4-thiazepine (0.55g) following the procedure of Description 2(a). This gave the title compound.

* J. Het. Chem. 3 , 466 (1966).

NMR (δ) ($CDCl_3$): 2.6 (2H,t), 2.75 (2H,t) 3.4-3.6 (5H,m), 7.4 (1H,dd), 7.65 (1H,dd), 8.5 (1H,dd).

e) 3,4-Dihydro-4-oxo-2H-pyrido[2,3-b]-1,4-thiazeoine-5-propanoic acid

This material is formed from methyl 3,4-dihydro-4-oxo-2H-pyrido[2,3-b]-1,4-thiazepine-5-propanoate, following the procedure of Description 7(d).

Description 20

a) Ethyl (3-nitro-2-pyridyl)oxyacetate

Sodium hydride (80% dispersion in mineral oil, 0.31g) was stirred under nitrogen in DMF (10 ml) as ethyl glycolate (1.0 ml) was added dropwise. After 10 min, 2-chloro-3-nitropyridine (1.65g) was added. After a further 15 min, the mixture was partitioned between ethyl acetate and water, and separated. The organic portion was washed with water and brine, dried ($Na_2SO_4$) and evaporated in vacuo . giving the title compound (2.1g) as a brown oil. $^1$H NMR revealed ca .20% contamination by the pyridine starting material (peaks not quoted below).
NMR (δ) ($CDCl_3$): 1.3 (3H,t), 4.25 (2H,q), 5.05 (2H,s), 7.1 (1H,m), 8.35 (2H,m).

b) Ethyl (3-amino-2-pyridyl)oxyacetate

This material was formed from ethyl (3-nitro-2-pyridyl) oxyacetate (2.09g), following the procedure of Description 3(c). This gave the title compound (1.76g) as a yellow oil.
NMR (δ) ($CDCl_4$): 1.3 (3H,t), 4.2 (2H,q), 4.9 (2H,s), 6.75 (1H,dd), 6.95 (1H,dd), 7.5 (1H,dd).

c) 2,3-Dihydro-3-oxo-4H-pyrido[2,3-b]-1,4-oxazine

Ethyl (3-amino-2-pyridyl)oxyacetate (1.75g) was stirred at reflux in dry xylene (50 ml) for 16h, evaporated to dryness, taken up in 10% methanol/chloroform and filtered. The filtrate was concentrated, and purified by chromatography on silica gel using methanol/chloroform (0-10% methanol, gradient). This gave the title compond (0.78g) as a brown solid.
NMR (δ) ($DMSOd_6$): 4.75 (2H,s), 7.0 (1H,ddd), 7.2 (1H,m), 7.8 (1H,m), 10.85 (1H,b).

d) 2,3-Dihydro-3-oxo-4H-pyrido[2,3-b]1,4-oxazine-4-propanoic acid

This material is formed from 2,3-dihydro-3-oxo-4 H -pyrido[2,3-b]-1,4-oxazine, following the procedures of Descriptions 2(a) and 7(d).

Description 21

a) 3 ((3-Nitro-4-pyridyl)thio)propanoic acid

This material was formed from 4-chloro-3-nitropyridine * (4.33g), following the procedure of Description 19(a). Some solid precipitated from the ethyl acetate extract, so this extract, still containing residual brine, was evaporated to dryness, washed with water, and dried. This gave the title compound (4.83g) as a brown powder.
NMR (δ) ($DMSOd_6$): 2.7 (2H,t), 3.3 (2H,t), 7.7 (1H,d), 8.65 (1H,d), 9.25 (1H,s), 12.55 (1H,b).

* J. Med. Chem. 32 (11), 2477 (1989).

b) 3,4-Dihydro-4-oxo-2H-pyrido[4,3-b]-1,4-thiazepine

This material is formed from 3-((3-nitro-4-pyridyl)thio)propanoic acid, following the procedures of Descriptions 19(b) and 19(c).

c) 3,4-Dihydro-4-oxo-2H-pyrido[4,3-b]-1,4-thiazeoine

This material is formed from 3,4-dihydro-4-oxo-2 H -pyrido[4,3-b]-1,4-thiazepine following the procedures of Descriptions 2(a) and 7(d).

Description 22

a) 2,3-Dihydro-3-oxo-4H-pyrido[4,3-b]-1,4-oxazine

This material is formed from 4-chloro-3-nitropyridine, following the procedures of Descriptions 20(a)-(c).

b) 2,3-Dihydro-3-oxo-4H-pyrido[4,3-b]-1,4-oxazine-4-propanoic acid

This material is formed from 2,3-dihydro-3-oxo-4 H -pyrido[4,3-b]-1,4-oxazine, following the procedures of Descriptions 2(a) and 7(d).

Example 1

2,3-Dihydrofuro[3,2-b]pyridine-3-acetyl-Phe-Leu-ACHPA isobutylamide.0.5$H_2O$

This material was formed from 2,3-dihydrofuro[3,2-b]pyridine-3-acetic acid (Description 1) (0.074g) and Phe-Leu-ACHPA isobutylamide (Description 5(c)) (0.15g), following the procedure of Description 3(a). The crude product was purified by chromatography on silica gel using methanol/chloroform (0-4% methanol, gradient) to afford the title compound (0.13g) as a cream solid.
NMR (δ) ($CD_3OD$): 0.7-1.9(29H,m), 2.2-2.5(3H,m), 2.7-3.25(5H,m), 3.6-4.1(4H,m), 4.2-4.45(2H,m), 4.5-4.75-(2H,m), 7.1-7.35(7H,m), 7.9(1H,m).
Analysis: $C_{39}H_{57}N_5O_6$.0.5$H_2O$ requires C, 66.8; H,8.3, N, 10.0%. Found: C,66.8; H,8.4; N,9.7%.
M.S. (m/z) (FAB) (M+1) = 692 (consistent with m.w. = 691).

Example 2

2,3-Dihydro-4-oxofuro[3,2-]pyridine-3-acetyl-Phe-Leu-ACHPA isobutylamide

To a stirred solution of 2,3-dihydrofuro[3,2-b]pyridine-3-acetyl-Phe-Leu-ACHPA isobutylamide (Example 1) (0.064g) in dry dichloromethane (1ml) at 0°C was added 80% 3-chloroperoxybenzoic acid (MCPBA) (0.020g) in three portions over 5 mins. After stirring at room temperature for 18h further 80% MCPBA (0.002g) was added and stirring continued for lh. The solvent was evaporated in vacuo and the residue purified by chromatography on silica gel using methanol/chloroform (0-6% methanol, gradient) to afford the title compound (0.058g) as a white solid.
M.S. (m/z) (FAB) (M+1) = 708 (consistent with m.w. = 707).

Example 3

2,3-Dihydro-3-oxo-4H-pyrido[3,2-b]-1,4-oxazine-4-propanoyl-Phe-Leu-ACHPA 3-(1-imidazolyl)propylamide .0.5($CHCl_3$)

This material was formed from 2,3-dihydro-3-oxo-4 H -pyrido[3,2-b]-1,4-oxazine-4-propanoic acid (Description 2(b)) (0.094g) and Phe-Leu-ACHPA 3-(1-imidazolyl)propylamide (Description 3(c)) (0.16g), following the procedure of Description 3(a). The crude product was purified by chromatography on silica gel using methanol/chloroform (0-11% methanol, gradient) to afford the title compound (0.186g) as a white solid.

NMR (δ) ($DMSOd_6$): 0.8-2.0(24H,m), 2.1(2H,bm), 2.3-3.1(6H,m), 3.8(2H,bm), 3.95(2H,t), 4.10(2H,bm), 4.3-(1H,m), 4.5(1H,bm), 4.7(2H,s), 4.9(1H,d), 6.9(1H,s), 7.0(1H,q), 7.1-7.5(8H,m), 7.6(1H,s), 7.8(1H,t), 8.0(1H,d), 8.1-8.3(2H,m), 8.3(0.5H,s)

Analysis: $C_{42}H_{58}N_8O_7.0.5CHCl_3$ requires C,59.6; H,7.0; N,13.2% Found: C,60.0; H,7.0; N,13.2%.

M.S. (m/z) (FAB) (M+1) = 787 (consistent with m.w. = 786).

## Example 4a

2,3-Dihydro-3-oxo-4H-pyrido[4,3-b]-1,4-thiazine-4-propanoyl-Phe-Leu-ACHPA 3-(1-imidazolyl)propylamide .$2H_2O$

This material was prepared from 2,3-dihydro-3-oxo-4 H -pyrido[4,3-b]-1,4-thiazine-4-propanoic acid (Description 6(b)) (0.11g), following the procedure of Example 3. Purification by chromatography on silica gel using methanol/chloroform (0-11% methanol, gradient) afforded the title compound (0.07g).

NMR (δ) ($DMSOd_6$): 0.7-1.9 (24H,m), 2.1 (2H,m), 2.4 (2H,m), 2.7-3.1 (4H,m), 3.55 (2H,s), 3.8-4.1 (1H,m), 4.9 (1H,d), 6.9 (1H,s), 7.1-7.3 (7H,m), 7.45 (1H,d), 7.6 (1H,s), 7.75 (1H,t), 8.1-8.2 (2H,m), 8.3 (1H,d), 8.7 (1H,s).

Analysis: $C_{42}H_{58}N_8O_6S.2H_2O$ requires C,60.1; H,7.5; N,13.4%. Found: C,60.1; H,7.1; N,13.0%.

M.S. (m/z) (FAB) (M+1) = 803 (consistent with m.w. = 802).

## Example 4b

2,3-Dihydro-3-oxo-4H-pyrido[4,3-b]-1,4-thiazine-4-propanoyl-Phe-Leu-ACHPA 3-(1-imidazolyl)propylamide .HCl

The corresponding free base (Example 4(a)) (0.03g) was dissolved in methanol (1 ml), acidified with a few drops of 5M hydrochloric acid, diluted with water (25 ml), and freeze-dried. This gave the title compound (0.03g) as a white powder.

NMR (δ) ($DMSOd_6$): 0.7-1.0 (8H,m), 1.0-1.45 (7H,m), 1.5-1.8 (7H,m), 1.9 (2H,m), 2.1 (2H,m), 2.45 (2H,m), 2.7 (1H,dd), 3.0 (3H,m), 3.95 (m), 4.05 (m), 4.2 (2H,t), 4.3 (1H,q), 4.55 (1H,m), 7.1-7.3 (5H,m), 7.4 (1H,d), 7.6 (1H,d), 7.7 (1H,s), 7.8 (1H,s), 7.85 (1H,t), 8.2 (2H,m), 8.35 (1H,d), 8.6 (1H,s), 9.1 (1H,s), 14.4 (b).

M.S. (m/z) (FAB) (M+1)=803 (consistent with m.w. of free base=802).

## Example 5

2,3-Dihydrofuro[3,2-b]pyridine-3-acetyl-Phe-Leu-ACHPA 3-(1-imidazolyl)propylamide

The material was prepared from 2,3-dihydrofuro[3,2-b]pyridine-3-acetic acid (Description 1(a)) (0.14g), following the procedure of Example 3. This gave the title compound (0.25g).

NMR (δ) ($DMSOd_6$): 0.7-1.9 (24H,m), 2.1 (2H,m), 2.3 (1H,m), 2.55-3.1 (5H,m), 3.7-4.0 (4H,m), 4.15-4.6 (3H,m), 4.7-4.9 (1H,bm), 6.9 (1H,s), 7.0-8.3 (14H,m).

M.S. (m/z) (FAB) (M+1) = 744 (consistent with m.w. = 743).

## Example 6

2,3-Dihydro-2-oxo-1H-imidazo[4,5-b]pyridine-3-propanoyl-Phe-Leu-ACHPA 3-(1-imidazolyl)propylamide.HCl

This material was formed from 2,3-dihydro-2-oxo-1 H -imidazo[4,5-b]pyridine-3-propanoic acid (Description 7(d)) (0.10g) and Phe-Leu-ACHPA 3-(1-imidazolyl)propylamide.2($CF_3CO_2H$) (Description 9(b)) (0.40g), following the coupling procedure of Description 3(b), but substituting N,N-diisopropylethylamine (DIPEA) for triethylamine. The crude product was purified by chromatography on silica gel using methanol/chloroform (0-10% methanol, gradient), giving the free base of the title compound. This was dissolved in methanol (2 ml), a slight excess of 5M hydrochloric acid was added, and the mixture was diluted with water (30 ml) and freeze-dried, giving the title compound (0.19g).

NMR (δ) ($DMSOd_6$): 0.7-1.0 (8H,m), 1.0-1.5 (7H,m), 1.5-1.85 (7H,m), 1.95 (2H,m), 2.15 (2H,m), 2.55 (2H,m), 2.8 (1H,m), 2.9-3.1 (3H,m), 3.8-4.0 (4H,m), 4.5 (1H,m), 7.0 (1H,dd), 7.1-7.4 (6H,m), 7.7 (1H,s), 7.8-8.0 (3H,m), 8.2 (1H,d), 8.3 (1H,m), 9.15 (1H,s), 11.15 (1H,s), 14.55 (1H,b).

M.S. (m/z) (FAB) (M+1) = 772 (consistent with m.w. of free base = 771).

Example 7

2,3-Dihydro-2-oxo-1H-imidazo[4,5-b]pyridine-1-propanoyl-Phe-Leu-ACHPA 3-(1-imidazolyl)propylamide.HCl

This material was formed from 2,3-dihydro-2-oxo-1 H - imidazo[4,5-b]pyridine-1-propanoic acid (Description 8(d)) (0.10g), following the procedure of Example 6. This gave the title compound (0.20g) as a white powder.

NMR (δ) ($DMSOd_6$): 0.7-1.8 (22H,m), 1.95 (2H,m), 2.1 (2H,m), 2.5 (m), 2.7 (1H,dd), 3.0 (3H,m), 3.85 (4H,m), 4.2 (m), 4.3 (m), 4.5 (1H,m), 6.95 (1H,dd), 7.1-7.4 (7H,m), 7.7 (1H,s), 7.8 (1H,s), 7.9 (2H,m), 8.2-8.4 (2H,m), 9.1 (1H,s), 11.5 (1H,b), 14.55 (1H,b).

M.S. (m/z) (FAB) (M+1)=772 (consistent with m.w. of free base=771).

Example 8

2,3-Dihydro-4-oxofuro[3.2-b]pyridine-3-acetyl-Phe-Leu-ACHPA 3-(1-imidazolyl)propylamide.2HCl

This material was formed from 2,3-dihydrofuro[3,2-b]pyridine-3-acetyl-Phe-Leu-ACHPA 3-(1-imidazolyl)-propylamide (Example 5) (0.31g), following the procedure of Example 2. After purification by chromatography, acidification and freeze-drying was performed as described in Example 6. This gave the title compound (0.12g).

NMR (δ) ($DMSOd_6$): 0.7-1.85 (22H,m), 1.9 (2H,m), 2.1 (2H,m), 2.25-2.45 (1H,m), 2.75 (1H,m), 3.05 (4H,m), 3.8-4.1 (3H,m), 4.2-4.4 (3H,m), 4.55 (2H,m), 6.9 (1H,m), 7.1-7.9 (12H,m), 8.2-8.5 (2H,m), 9.15 (1H,s), 14.7 (1H,b).

M.S. (m/z) (FAB) (M+1)=760 (consistent with m.w. of free base=759).

Example 9

2,3-Dihydro-1,1,3-trioxo-4H-pyrido[4,3-b]-1,4-thiazine4-propanoyl-Phe-Leu-ACHPA 3-(1-imidazolyl)-propylamide .HCl

This material was formed from 2,3-dihydro-1,1,3-trioxo-4 H -pyrido[4,3-b]-1,4-thiazine-4-propanoic acid (Descriptiion 10(c)) (0.058g), following the procedure of Example 6. This gave the title compound (0.098g) as a fluffy white powder.

NMR (δ) ($DMSOd_6$): 0.7-1.0 (8H,m), 1.0-1.5 (7H,m), 1.5-1.8 (7H,m), 1.9 (2H,m), 2.1 (2H,m), 2.45 (2H,m), 2.7 (1H,dd), 3.05 (3H,m), 3.85 (2H,bs), 4.0-4.25 (4H,m), 4.3 (1H,q), 4.6 (1H,m), 4.95 (2H,m), 7.1-7.3 (6H,m), 7.35 (1H,d), 7.7 (1H,s), 7.8 (1H,s), 7.85 (2H,m), 8.25 (1H,d), 8.4 (1H,d), 8.65 (1H,d), 8.95 (1H,s), 9.1 (1H,s), 14.45 (1H,b).

M.S. (m/z) (FAB)(M+1)=835 (consistent with m.w. of free base=834).

Example 10

2,3-Dihydro-3-oxo-4H-pyrido[2,3-b]-1,4-thiazine-4-propanoyl-Phe-Leu-ACHPA 3-(1-imidazolyl)propylamide .HCl

This material was formed from 2,3-dihydro-3-oxo-4 H -pyrido[2,3-b]-1,4-thiazine-4-propanoic acid (Description 11(c)) (0.12g), following the procedure of Example 6. This gave the title compound (0.21g) as a white solid.

NMR (δ) ($DMSOd_6$): 0.7-1.0 (8H,m), 1.0-1.5 (7H,m), 1.5-1.8 (7H,m), 1.9 (2H,m), 2.1 (2H,m), 2.4 (2H,m), 2.7 (1H,dd), 3.05 (3H,m), 3.65 (2H,s), 3.85 (3H,m), 4.0 (1H,m), 4.2 (2H,t), 4.3 (1H,q), 4.55 (1H,m), 7.1-7.3 (6H,m), 7.45 (1H,d), 7.7 (2H,m), 7.8 (1H,s), 7.9 (1H,m), 8.15 (1H,d), 8.25 (1H,d), 8.4 (1H,d), 9.15 (1H,s), 14.65 (1H,b).

M.S. (m/z) (FAB) (M + 1) = 803 (consistent with m.w. of free base = 802).

Example 11

2,3-Dihydro-1,1,3-trioxo-4H-pyrido[2,3-b]-1,4-thiazine4-propanoyl-Phe-Leu-ACHPA 3-(1-imidazolyl)-propylamide .HCl.1.5$H_2O$

This material was formed from 2,3-dihydro-1,1,3-trioxo-4 H -pyrido[2,3-b]-1,4-thiazine-4-propanoic acid (Description 12(b)) (0.15g), following the procedure of Example 6. This gave the title compound (0.23g) as a white solid.

NMR (δ) ($DMSOd_6$): 0.7-1.0 (8H,m), 1.0-1.5 (7H,m), 1.5-1.8 (7H,m), 1.9 (2H,m), 2.15 (2H,m), 2.4 (2H,m), 2.75 (1H,dd), 3.05 (3H,m), 3.85 (2H,bs), 3.95 (1H,m), 4.05 (1H,m), 4.2 (2H,t), 4.3 (1H,q), 4.55 (1H,m), 4.9 (2H,m), 7.1-7.3 (6H,m), 7.4 (1H,d), 7.7 (1H,s), 7.75-7.9 (3H,m), 8.05 (1H,m), 8.25 (1H,d), 8.4 (1H,d), 8.5 (1H,d), 9.1 (1H,s), 14.5 (1H,b).

Analysis: $C_{42}H_{58}N_8O_8S$.HCl.1.5$H_2O$ requires C,56.1; H,7.0; N,12.5%. Found: C,56.0; H,6.6; N,12.2%.

M.S. (m/z) (FAB) (M + 1) = 835 (consistent with m.w. of free base = 834).

Example 12

6,7-Dihydro-5-oxo-5H-pyrrolo[3,4-b]pyridine-6-butanoyl-Phe-Leu-ACHPA 3-(1-imidazolyl)propylamide.HCl

This material was formed from 6,7-dihydro-5-oxo-5 H -pyrrolo[3,4-b]pyridine-6-butanoic acid (Description 13(d)) (0.15g), following the procedure of Example 6. This gave the title compound (0.14g).

NMR (δ) ($DMSOd_6$): 0.6-1.0 (8H,m), 1.0-1.4 (6H,m), 1.5-1.8 (8H,m), 1.9 (2H,t), 2.1 (3H,m), 2.7-3.1 (3H,m), 3.4 (1H,m), 3.8 (2H,m), 4.1-4.5 (5H,m), 4.5-5.4 (m), 7.1-7.6 (7H,m), 7.6-8.3 (6H,m), 8.7 (1H,d), 9.1 (1H,s), 14.6 (1H,b).

M.S. (m/z) (FAB) (M + 1) = 785 (consistent with m.w. of free base = 784).

Example 13

6,7-Dihydro-7-oxo-5H-pyrrolo[3,4-b]pyridine-6-butanoyl-Phe-Leu-ACHPA 3-(1-imidazolyl)propylamide.HCl

This material was formed from 6,7-dihydro-7-oxo-5 H -pyrrolo[3,4-b]pyridine-6-butanoic acid (Description 14(c)) (0.12g), following the procedure of Example 6. This gave the tit.le compound (0.09g).

NMR (δ) ($DMSOd_6$): 0.7-1.0 (6H,m), 1.0-1.4 (6H,m), 1.4-1.8 (8H,m), 1.9 (2H,t), 2.0-2.1 (3H,m), 2.7 (1H,t), 2.9-3.1 (3H,m), 3.4-3.5 (1H,m), 3.6 (2H,m), 4.1-4.6 (6H,m), 4.6-5.1 (m), 7.1-7.4 (6H,m), 7.6 (1H,m), 7.7 (1H,s), 7.8 (1H,s), 7.9 (1H,t), 8.05 (1H,d), 8.2 (2H,m), 8.7 (1H,d), 9.1 (1H,s), 14.6 (1H,s).

M.S. (m/z) (FAB) (M + 1) = 785 (consistent with m.w. of free base = 784).

Example 14

6,7-Dihydro-5H-7-oxo-pyrrolo[3,4-c]pyridine-6-butanoyl-Phe-Leu-ACHPA 3-(1-imidazolyl)propylamide.2HCl

This material was formed from 6,7-dihydro-5 H -7-oxo-pyrrolo[3,4-c]pyridine-6-butanoic acid.HCl (Description 15(c)) (0.19g), following the procedure of Example 6. This gave the title compound (0.28g).
NMR (δ) ($DMSOd_6$): 0.65-1.0 (8H,m), 1.0-1.5 (8H,m), 1.5-1.8 (8H,m), 1.9 (2H,t), 2.0-2.2 (4H,m), 2.7-2.8 (1H,m), 2.9-3.1 (3H,m), 3.4 (2H,m), 3.8 (2H,s), 4.15-4.3 (3H,m), 4.5 (1H,m), 4.65 (2H,m), 4.9-5.9 (3H,m), 7.1-7.5 (6H,m), 7.6-8.3 (6H,m), 8.9 (1H,d), 9.05-9.2 (2H,m), 14.6 (1H,bs).
M.S. (m/z) (FAB) (M+1)=785 (consistent with m.w. of free base=784).

Example 15

7-Oxo-5,6,7,8-tetrahydro-8H-1,8-naphthyridine-8-butanoyl-Phe-Leu-ACHPA 3-(1-imidazolyl)propylamide.HCl

This material was formed from 7-oxo-5,6,7,8-tetrahydro-8H-1,8-naphthyridine-8-butanoic acid (Description 16(b)) (0.06g), following the procedure of Example 6. This gave the title compound (0.10g).
NMR (δ) ($DMSOd_6$): 0.65-1.0 (8H,m), 1.0-1.75 (16H,m), 1.9 (2H,m), 2.0-2.2 (4H,m), 2.6 (2H,dd), 3.8 (2H,m), 3.9 (2H,m), 4.2 (4H,m), 4.5 (1H,m), 7.0 (1H,m), 7.2 (5H,m), 7.3-7.55 (1H,m), 7.6 (1H,d), 7.7 (1H,s), 7.8 (1H,s), 7.9 (1H,m), 8.1-8.3 (3H,m), 9.1 (1H,s), 14.5 (1H,b).
M.S. (m/z) (FAB) (M+1)=799 (consistent with m.w. of free base=798).

Example 16

6-Oxo-5,6,7,8-tetrahydro-5H-1,5-naphthyridine-5-butanoyl-Phe-Leu-ACHPA 3-(1-imidazolyl)propylamide.HCl

This material was formed from 6-oxo-5,6,7,8-tetrahydro-5 H -1,5-naphthyridine-5-butanoic acid (Description 17(b)) (0.12g), following the procedure of Example 6. This gave the title compound (0.23g).
NMR (δ) ($DMSOd_6$): 0.7-0.95 (8H,m), 1.0-1.8 (16H,m), 1.9 (2H,m), 2.1 (4H,m), 2.8 (2H,m), 3.0 (2H,m), 3.35 (2H,m), 3.8 (2H,b), 4.2 (2H,m), 4.55 (1H,m), 7.0-7.3 (6H,m), 7.4-7.6 (1H,m), 7.65 (1H,s), 7.8 (2H,m), 7.85-8.1 (2H,m), 8.15-8.4 (3H,m), 9.15 (1H,d).
M.S. (m/z) (FAB) (M+1)=799 (consistent with m.w. of free base=798).

Example 17a

7-Oxo-4,5,6,7-tetrahydro-6H-thieno[2,3-c]pyridine-6-pentanoyl-Phe-Leu ACHPA 3-(1-imidazolyl)-propylamide.$H_2O$

This material is formed from 7-oxo-4,5,6,7-tetrahydro-6H-thieno[2,3-c]pyridine-6-pentanoic acid (Description 18(b)) following the coupling procedure of Example 6. The crude product is purified by column chromatography on silica gel using methanol/chloroform (0-10% methanol/chloroform, gradient) to give the title compound.
NMR (δ) ($DMSOd_6$): 0.7-1.9 (29H,m), 2.0-2.2 (4H,m), 2.7-3.2 (6H,m), 3.5 (2H,m), 3.8 (2H,m), 3.9 (2H,m), 4.3 (1H,m), 4.5 (1H,m), 4.8-4.9 (1H,m), 6.9 (1H,d), 7.0 (1H,dd), 7.1 (2H,m), 7.2-7.3 (5H,m), 7.5 (1H,d), 7.6 (1H,d), 7.7 (2H,m), 8.0-8.2 (2H,m).
M.S. (m/z) (FAB) (M+1)=818 (consistent with m.w.=817).
Analysis: $C_{44}H_{63}N_7O_6S.H_2O$ requires C,63.2; H,7.8; N,11.7%. Found: C,63.2; H,7.8; N,11.8%.

Example 17b

7-Oxo-4,5,6,7-tetrahydro-6H-thieno[2,3-c]pyridine-6-pentanoyl-Phe-Leu-ACHPA 3-(1-imidazolyl)propylamide .HCl.1.5$H_2O$

This material is formed from the corresponding free base (Example 17(a)) following the procedure of Example 4(b).

NMR (δ) (DMSOd6): 0.7-1.9 (29H,m), 2.1-2.3 (5H,m), 2.7-3.1 (5H,m), 3.3-3.4 (2H,m), 3.5 (2H,q), 3.9 (2H,m), 4.2-4.6 (3H,m), 7.0 (1H,m), 7.1 (1H,m), 7.2-7.3 (5H,m), 7.4 (1H,d), 7.6 (1H,d), 7.7 (1H,s), 7.7-7.8 (2H,m), 7.9 (1H,t), 8.0-8.3 (2H,m), 9.1 (1H,s), 14.5 (1H,bs).

Analysis: $C_{44}H_{63}N_7O_6S$.HCl .1.5$H_2O$ requires C,59.9; H,7.6; N,11.1%. Found: C,59.8; H,7.6; N,11.2%.

Example 18

3,4-Dihydro-4-oxo-2H-pyrido[2,3-b]-1,4-thiazepine-5-propanoyl-Phe-Leu-ACHPA 3-(1-imidazolyl)propylamide

This material is formed from 3,4-dihydro-4-oxo-2 H -pyrido[2,3-b]-1,4-thiazepine-5-propanoic acid (Description 19(e)), following the procedure of Example 3.

Example 19

2,3-Dihydro-3-oxo-4H-pyrido[2,3-b]-1,4-oxazine-4-propanoyl-Phe-Leu-ACHPA 3-(1-imidazolyl)propylamide

This material is formed from 2,3-dihydro-3-oxo-4 H -pyrido[2,3-b]-1,4-oxazine-4-propanoic acid (Description 20(d)), following the procedure of Example 3.

Example 20

3,4-Dihydro-4-oxo-2H-pyrido[4,3-b]-1,4-thiazepine-5-propanoyl-Phe-Leu-ACHPA 3-(1-imidazolyl)propylamide

This material is formed from 3,4-dihydro-4-oxo-2 H pyrido[4,3-b]-1,4-thiazepine-5-propanoic acid (Description 21(c)), following-the procedure of Example 3.

Example 21

2,3-Dihydro-3-oxo-4H-pyrido[4,3-b]-1,4-oxazine-4-propanoyl-Phe-Leu-ACHPA 3-(1-imidazolyl)propylamide

This material is formed from 2,3-dihydro-3-oxo-4 H -pyrido[4,3-b]-1,4-oxazine-4-propanoic acid (Description 22(b)), following the procedure of Example 3.

Biological Data

In vitro human renin inhibition

Renin inhibitory activity was estimated as the percentage change in renin activity in human plasma in the presence and absence of compound. The source of plasma was blood taken from healthy volunteers. Renin activity was defined by the difference in angiotensin I levels between two halves of a sample, one incubated at 37° and the other at 4° for 2 h. Angiotensin I levels were measured using a $^{125}$I- angiotensin I radioimmunoassay kit (NEN/Dupont, Stevenage). Results were calculated as the mean of at least two, duplicate determinations and $IC_{50}$ values were calculated by linear regression analysis of at least three

concentrations of compound.

The results were as follows:

| Compound | $IC_{50}$(n-m) |
|---|---|
| E2 | 21 |
| E3 | 26 |
| E4 | 4 |
| E10 | 6 |
| E11 | 12 |
| E14 | 14 |

**Claims**

1. A compound of formula (I), or a pharmaceutically acceptable salt thereof:

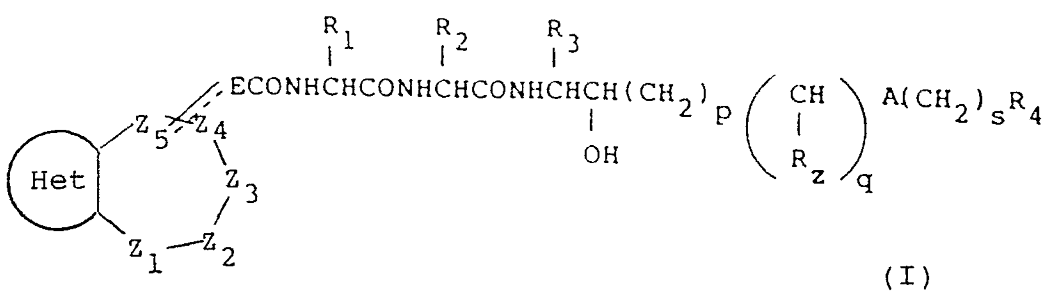

(I)

wherein the Het ring is of sub-formula (a) or (b):

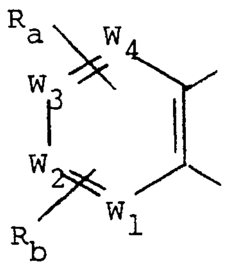

(a)

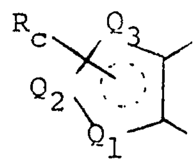

(b)

wherein
one or two of $W_1$, $W_2$, $W_3$ and $W_4$ is N or NO (such that if two are NO then these are are $W_1$ and $W_4$), and the others are CH, $CR_a$ or $CR_b$;
one of $Q_1$, $Q_2$ and $Q_3$ is S and the other two are CH and $CR_c$;
either $Z_1$ and $Z_2$ are absent and $Z_3$, $Z_4$ and $Z_5$ and the carbon atoms to which $Z_3$ and $Z_5$ are attached, form a 5-membered non-aromatic heterocyclic ring; or
$Z_1$ is absent and $Z_2$, $Z_3$, $Z_4$, $Z_5$ and the carbon atoms to which $Z_2$ and $Z_5$ are attached, form a 6-membered non-aromatic heterocyclic ring; or
$Z_1$, $Z_2$, $Z_3$, $Z_4$ and $Z_5$ and the carbon atoms to which $Z_1$ and $Z_5$ are attached, form a 7-membered non-aromatic heterocyclic ring;
E is absent or is $(CH_2)n$ or CH(CH2)n-1 wherein n is 1 to 4;
A is -CONH-, -NHCO-, -COO-, $-S(O)_r-$ wherein r is 0, 1 or 2, or $-CH_2-$;
p is 0, 1 or 2;
s is 0, 1, 2, 3 or 4;
q is 0 or 1;
$R_z$ is hydrogen, $C_{1-6}$ alkyl or, when A is $-CH_2-$, hydroxy;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl, $C_{3-8}$ cycloalkyl or phenyl;
$R_4$ is $C_{1-6}$ alkyl, $C_{3-8}$ cycloalkyl, a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, or $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and
the dashed line represents an optional bond (when E is present).
2. A compound according to claim 1 wherein $Z_1$ and $Z_2$ are absent, $Z_3$ is CO, $Z_4$ is N, $Z_5$ is CH and E is attached at $Z_4$.
3. A compound according to claim 1 wherein the Het ring is of sub-formula (a), $W_3$ is N and $W_1$, $W_2$ and $W_4$ are CH, $CR_a$ or $CR_b$, $Z_1$ is absent, $Z_2$ is S, $Z_3$ is $CH_2$, $Z_4$ is CO, $Z_5$ is N and E is attached at $Z_5$.
4. A compound according to any one of claims 1 to 3 wherein one of $R_a$ and $R_b$ in sub-formula (b) is hydrogen and the other is $CH_2NH_2$, $CO_2H$ or $CH_2NHCH_2CO_2H$, or $R_c$ in sub-formula (b) is hydrogen.
5. A compound according to any one of claims 1 to 4 wherein the amino acid residue containing $R_2$ is Leu,

β-pyrazolylalanine or His.

6. A compound according to claim 1 of formula (IA) or a pharmaceutically acceptable salt thereof:

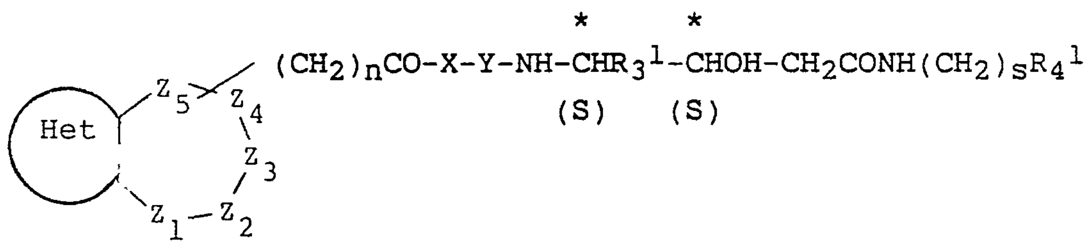

(IA)

wherein

X is Phe;

Y is Leu or His;

$R_3{}^1$ is cyclohexylmethyl;

$R_4$ is $C_{4-5}$ alkyl and s is 0 or s is 2 to 4 and $R_4{}^1$ is carboxy or a saturated or unsaturated heterocyclic ring; and

the remaining variables are as defined in claim 1.

7. A compound according to any one of claims 1 to 6 wherein E is $(CH_2)_n$ and E is attached at $Z_4$ or $Z_5$.

8. A compound according to any one of claims 1 to 7 wherein the sequence from the amino acid containing $R_3$ or $R_3{}^1$ to $NH(CH_2)_sR_4$, is 4(S)-amino-5-cyclohexyl-3(S)-hydroxypentanoic acid (ACHPA).

9. A compound according to any one of claims 1 to 8 wherein s is 0 and $R_4$ is isobutyl or s is 2, 3 or 4 and $R_4$ is 1-imidazolyl, 2-imidazolyl, 4-morpholinyl, 2-oxopyrrolidin-1-yl, 2-pyridyl, 2-pyridyl-N-oxide, 3-pyridyl, hydroxyethyl, acetylamino, 4-methylpiperazin-1-yl, piperazin-1-yl or dimethylamino.

10. A compound selected from the group consisting of:

2,3-dihydrofuro[3,2-b]pyridine-3-acetyl-Phe-Leu-ACHPA isobutylamide,

2,3-dihydro-4-oxofuro[3,2-b]pyridine-3-acetyl-Phe-Leu-ACHPA isobutylamide,

2,3-dihydro-3-oxo-4 H -pyrido[3,2-b]-1,4-oxazine-4-propanoyl-Phe-Leu-ACHPA 3-(1-imidazolyl)propylamide,

2,3-dihydro-3-oxo-4 H -pyrido[4,3-b]-1,4-thiazine-4-propanoyl-Phe-Leu-ACHPA 3-(1-imidazolyl)-propylamide,

2,3-dihydrofuro[3,2-b]pyridine-3-acetyl-Phe-Leu-ACHPA 3-(1-imidazolyl)propylamide,

2,3-dihydro-2-oxo-1 H -imidazo[4,5-b]pyridine-3-propanoyl-Phe-Leu-ACHPA 3-(1-imidazolyl)propylamide,

2,3-dihydro-2-oxo-1 H -imidazo[4,5-b]pyridine-1-propanoyl-Phe-Leu-ACHPA 3-(1-imidazolyl)propylamide,

2,3-dihydro-4-oxofuro[3,2-b]pyridine-3-acetyl-Phe-Leu-ACHPA 3-(1-imidazolyl)propylamide,

2,3-dihydro-1,1,3-trioxo-4 H -pyrido[4,3-b]-1,4-thiazine-4-propanoyl-Phe-Leu-ACHPA 3-(1-imidazolyl)-propylamide, 2,3-dihydro-3-oxo-4 H -pyrido[2,3-b]-1,4-thiazine-4-propanoyl-Phe-Leu-ACHPA 3-(1-imidazolyl)propylamide, 2,3-dihydro-1,1,3-trioxo-4 H -pyrido[2,3-b]-1,4-thiazine-4-propanoyl-Phe-Leu-ACHPA 3-(1-imidazolyl)-propylamide,

6,7-dihydro-5-oxo-5 H -pyrrolo[3,4-b]pyridine-6-butanoyl-Phe-Leu-ACHPA 3-(1-imidazolyl)propylamide,

6,7-dihydro-7-oxo-5 H -pyrrolo[3,4-b]pyridine-6-butanoyl-Phe-Leu-ACHPA 3-(1-imidazolyl)propylamide,

6,7-dihydro-5 H -7-oxo-pyrrolo[3,4-c]pyridine-6-butanoyl-Phe-Leu-ACHPA 3-(1-imidazolyl)propylamide,

7-oxo-5,6,7,8-tetrahydro-8 H -1,8-naphthyridine-8-butanoyl-Phe-Leu-ACHPA 3-(1-imidazolyl)propylamide,

6-oxo-5,6,7,8-tetrahydro-5 H -1,5-naphthyridine-5-butanoyl-Phe-Leu-ACHPA 3-(1-imidazolyl)propylamide,

7-oxo-4,5,6,7-tetrahydro-6 H -thieno[2,3-c]-pyridine-6-pentanoyl-Phe-Leu-ACHPA 3-(1-imidazolyl)-propylamide,

3,4-dihydro-4-oxo-2 H -pyrido[2,3-b]-1,4-thiazepine-5-propanoyl-Phe-Leu-ACHPA 3-(1-imidazolyl)-propylamide,

2,3-dihydro-3-oxo-4 H -pyrido[2,3-b]-1,4-oxazine-4-propanoyl-Phe-Leu-ACHPA 3-(1-imidazolyl)propylamide,

3,4-dihydro-4-oxo-2 H -pyrido[4,3-b]-1,4-thiazepine-5-propanoyl-Phe-Leu-ACHPA 3-(1-imidazolyl)-propylamide and

2,3-dihydro-3-oxo-4 H -pyrido[4,3-b]-1,4-oxazine-4-propanoyl-Phe-Leu-ACHPA 3-(1-imidazolyl)propylamide.

11. A process for the preparation of a compound according to claim 1, which process comprises reacting a reagent of the formula (III):

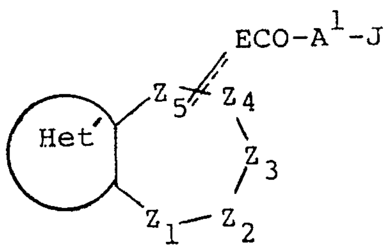

(III)

wherein Het′ is Het as defined in claim 1 but wherein $R_a$, $R_b$ and $R_c$ are $R_a'$, $R_b'$ and $R_c'$, which are $R_a$, $R_b$ and $R_c$ respectively or groups or atoms convertible thereto; $A^1$ is absent or represents an appropriate amino acid or dipeptide unit; J is OH or a leaving group; and the remaining variables are as defined in claim 1; with a reagent of the formula (IV):

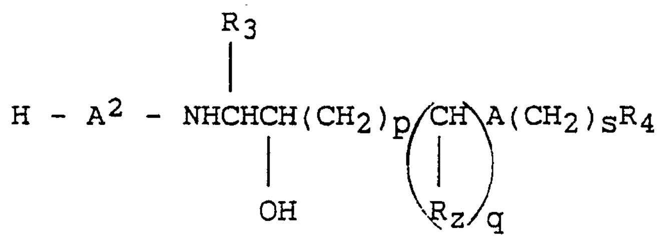

(IV)

wherein
$A^2$ is absent or represents an appropriate amino acid or dipeptide unit, such that $A^1$ + $A^2$ is $-NHCHR_1CONHCHR_2CO-$, and the remaining variables are as defined in claim 1; and thereafter, if desired or necessary deprotecting (within $A^1$ or $A^2$) of the products, and/or converting $Z_1$, $Z_2$, $Z_3$, $Z_4$ or $Z_5$ to other $Z_1$, $Z_2$, $Z_3$, $Z_4$ or $Z_5$, $R_a'/R_b'/R_c'$ to $R_a$, $R_b$ and/or $R_c$ and/or forming a pharmaceutically acceptable salt thereof.

12. A pharmaceutical composition comprising a compound according to any one of claims 1 to 10, and a pharmaceutically acceptable carrier.

13. A compound according to any one of claims 1 to 10 for use as an active therapeutic substance.

14. A compound according to any one of claims 1 to 10 for use in the treatment of hypertension.

15. Use of a compound according to any one of claims 1 to 10 in the manufacture of a medicament for use in the treatment of hypertension.

Claims for the following Contracting State: ES.

1. A process for the preparation of a compound of formula (I), or a pharmaceutically acceptable salt thereof:

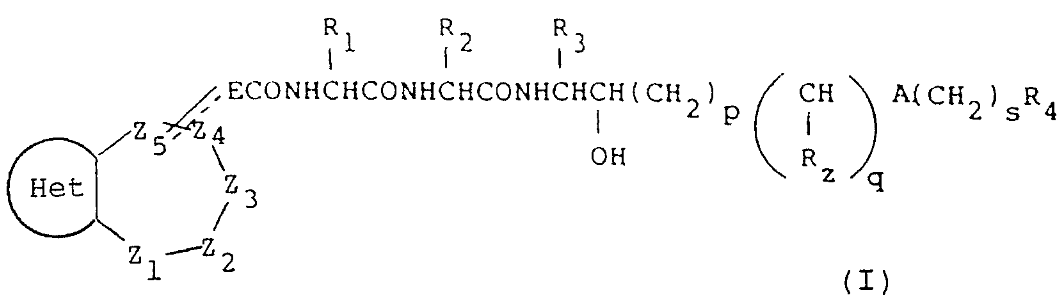

(I)

wherein
the Het ring is of sub-formula (a) or (b):

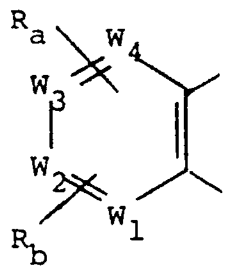

(a)

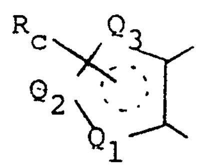

(b)

wherein
one or two of $W_1$, $W_2$, $W_3$ and $W_4$ is N or NO (such that if two are NO then these are are $W_1$ and $W_4$), and the others are CH, $CR_a$ or $CR_b$;
one of $Q_1$, $Q_2$ and $Q_3$ is S and the other two are CH and $CR_c$;
either $Z_1$ and $Z_2$ are absent and $Z_3$, $Z_4$ and $Z_5$ and the carbon atoms to which $Z_3$ and $Z_5$ are attached, form a 5-membered non-aromatic heterocyclic ring; or
$Z_1$ is absent and $Z_2$, $Z_3$, $Z_4$, $Z_5$ and the carbon atoms to which $Z_2$ and $Z_5$ are attached, form a 6-membered non-aromatic heterocyclic ring; or
$Z_1$, $Z_2$, $Z_3$, $Z_4$ and $Z_5$ and the carbon atoms to which $Z_1$ and $Z_5$ are attached, form a 7-membered non-aromatic heterocyclic ring;
E is absent or is $(CH_2)_n$ or $CH(CH_2)_{n-1}$ wherein n is 1 to 4;
A is -CONH-, -NHCO-, -COO-, $-S(O)_r-$ wherein r is 0, 1 or 2, or $-CH_2-$;
p is 0, 1 or 2;
s is 0, 1, 2, 3 or 4;
q is 0 or 1;
$R_z$ is hydrogen, $C_{1-6}$ alkyl or, when A is $-CH_2-$, hydroxy;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-5}$ alkoxycarbonylamino;
$R_3$ is $CH_2R_{12}$ wherein $R_{12}$ is $C_{1-6}$ alkyl, $C_{3-8}$ cycloalkyl or phenyl;
$R_4$ is $C_{1-6}$ alkyl, $C_{3-8}$ cycloalkyl, a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, or $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and
the dashed line represents an optional bond (when E is present);
which process comprises reacting a reagent of the formula (III):

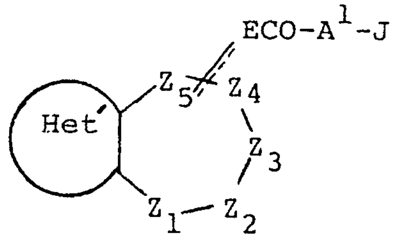

(III)

wherein Het′ is Het as defined in claim 1 but wherein $R_a$, $R_b$ and $R_c$ are $R_a'$, $R_b'$ and $R_c'$, which are $R_a$, $R_b$ and $R_c$ respectively or groups or atoms convertible thereto; $A^1$ is absent or represents an appropriate amino acid or dipeptide unit; J is OH or a leaving group; and the remaining variables are as defined with a reagent of the formula (IV):

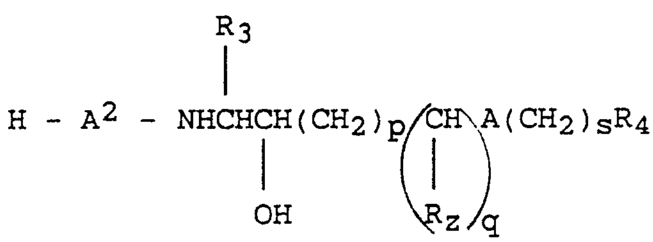

(IV)

wherein

$A^2$ is absent or represents an appropriate amino acid or dipeptide unit, such that $A^1 + A^2$ is $-NHCHR_1CONHCHR_2CO-$, and the remaining variables are as defined and thereafter, if desired or necessary deprotecting (within $A^1$ or $A^2$) of the products, and/or converting $Z_1$, $Z_2$, $Z_3$. $Z_4$ or $Z_5$ to other $Z_1$, $Z_2$, $Z_3$, $Z_4$ or $Z_5$, $R_a'/R_b'/R_c'$ to $R_a$, $R_b$ and/or $R_c$ and/or forming a pharmaceutically acceptable salt thereof.

2. A process according to claim 1 wherein $Z_1$ and $Z_2$ are absent, $Z_3$ is CO, $Z_4$ is N, $Z_5$ is CH and E is attached at $Z_4$.

3. A process according to claim 1 wherein the Het ring is of sub-formula (a), $W_3$ is N and $W_1$, $W_2$ and $W_4$ are CH, $CR_a$ or $CR_b$, $Z_1$ is absent, $Z_2$ is S, $Z_3$ is $CH_2$, $Z_4$ is CO, $Z_5$ is N and E is attached at $Z_5$.

4. A process according to any one of claims 1 to 3 wherein one of Ra and Rb in sub-formula (b) is hydrogen and the other is $CH_2NH_2$, $CO_2H$ or $CH_2NHCH_2CO_2H$, or $R_c$ in sub-formula (b) is hydrogen.

5. A process according to any one of claims 1 to 4 wherein the amino acid residue containing $R_2$ is Leu, β-pyrazolylalanine or His.

6. A process according to claim 1 for the preparation of a compound of formula (IA) or a pharmaceutically acceptable salt thereof:

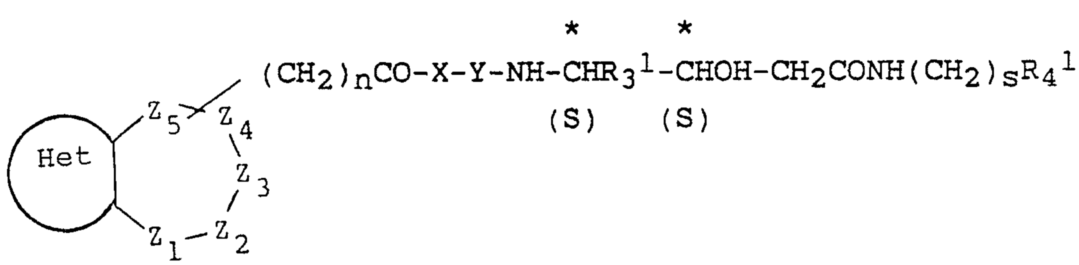

(IA)

wherein
X is Phe;
Y is Leu or His;
$R_3'$ is cyclohexylmethyl;
$R_4'$ is $C_{4-5}$ alkyl and s is 0 or s is 2 to 4 and $R_4'$ is carboxy or a saturated or unsaturated heterocyclic ring; and
the remaining variables are as defined in claim 1.

7. A process according to any one of claims 1 to 6 wherein E is $(CH_2)_n$ and E is attached at $Z_4$ or $Z_5$.

8. A process according to any one of claims 1 to 7 wherein the sequence from the amino acid containing $R_3$ or $R_3'$ to $NH(CH_2)_sR_4$, is 4(S)-amino-5-cyclohexyl-3(S)-hydroxypentanoic acid (ACHPA).

9. A process according to any one of claims 1 to 8 wherein s is 0 and $R_4$ is isobutyl or s is 2, 3 or 4 and $R_4$ is 1-imidazolyl, 2-imidazolyl, 4-morpholinyl, 2-oxopyrrolidin-1-yl, 2-pyridyl, 2-pyridyl-N-oxide, 3-pyridyl, hydroxyethyl, acetylamino, 4-methylpiperazin-1-yl, piperazin-1-yl or dimethylamino.

10. A process for the preparation of a compound selected from the group consisting of:
2,3-dihydrofuro[3,2-b]pyridine-3-acetyl-Phe-Leu-ACHPA isobutylamide,
2,3-dihydro-4-oxofuro[3,2-b]pyridine-3-acetyl-Phe-Leu-ACHPA isobutylamide,
2,3-dihydro-3-oxo-4 H -pyrido[3,2-b]-1,4-oxazine-4-propanoyl-Phe-Leu-ACHPA 3-(1-imidazolyl)propylamide,
2,3-dihydro-3-oxo-4 H -pyrido[4,3-b]-1,4-thiazine-4-propanoyl-Phe-Leu-ACHPA 3-(1-imidazolyl)-propylamide,
2,3-dihydrofuro[3,2-b]pyridine-3-acetyl-Phe-Leu-ACHPA 3-(1-imidazolyl)propylamide,
2,3-dihydro-2-oxo-1 H -imidazo[4,5-b]pyridine-3-propanoyl-Phe-Leu-ACHPA 3-(1-imidazolyl)propylamide,
2,3-dihydro-2-oxo-1 H -imidazo[4,5-b]pyridine-1-propanoyl-Phe-Leu-ACHPA 3-(1-imidazolyl)propylamide,
2,3-dihydro-4-oxofuro[3,2-b]pyridine-3-acetyl-Phe-Leu-ACHPA 3-(1-imidazolyl)propylamide,
2,3-dihydro-1,1,3-trioxo-4 H -pyrido[4,3-b]-1,4-thiazine-4-propanoyl-Phe-Leu-ACHPA 3-(1-imidazolyl)-propylamide,
2,3-dihydro-3-oxo-4 H -pyrido[2,3-b]-1,4-thiazine-4-propanoyl-Phe-Leu-ACHPA 3-(1-imidazolyl)propylamide,
2,3-dihydro-1,1,3-trioxo-4 H -pyrido[2,3-b]-1,4-thiazine-4-propanoyl-Phe-Leu-ACHPA 3-(1-imidazolyl)-propylamide,
6,7-dihydro-5-oxo-5 H -pyrrolo[3,4-b]pyridine-6-butanoyl-Phe-Leu-ACHPA 3-(1-imidazolyl)propylamide,
6,7-dihydro-7-oxo-5 H -pyrrolo[3,4-b]pyridine-6-butanoyl-Phe-Leu-ACHPA 3-(1-imidazolyl)propylamide,
6,7-dihydro-5 H -7-oxo-pyrrolo[3,4-c]pyridine-6-butanoyl-Phe-Leu-ACHPA 3-(1-imidazolyl)propylamide,
7-oxo-5,6,7,8-tetrahydro-8 H -1,8-naphthyridine-8-butanoyl-Phe-Leu-ACHPA 3-(1-imidazolyl)propylamide,
6-oxo-5,6,7,8-tetrahydro-5 H -1,5-naphthyridine-5-butanoyl-Phe-Leu-ACHPA 3-(1-imidazolyl)propylamide,
7-oxo-4,5,6,7-tetrahydro-6 H -thieno[2,3-c]-pyridine-6-pentanoyl-Phe-Leu-ACHPA 3-(1-imidazolyl)-propylamide,
3,4-dihydro-4-oxo-2 H -pyrido[2,3-b]-1,4-thiazepine-5-propanoyl-Phe-Leu-ACHPA 3-(1-imidazolyl)-propylamide,
2,3-dihydro-3-oxo-4 H -pyrido[2,3-b]-1,4-oxazine-4-propanoyl-Phe-Leu-ACHPA 3-(1-imidazolyl)propylamide,
3,4-dihydro-4-oxo-2 H -pyrido[4,3-b]-1,4-thiazepine-5-propanoyl-Phe-Leu-ACHPA 3-(1-imidazolyl)-propylamide and
2,3-dihydro-3-oxo-4 H -pyrido[4,3-b]-1,4-oxazine-4-propanoyl-Phe-Leu-ACHPA 3-(1-imidazolyl)propylamide.

11. Use of a compound prepared according to a process in any one of claims 1 to 10, in the manufacture of a medicament for use in the treatment of hypertension.